# EUROPEAN PATENT SPECIFICATION

(11) **EP 1 896 026 B1**
(45) Date of publication and mention of the grant of the patent: **09.10.2013**
(21) Application number: 06763345.3
(22) Date of filing: 30.05.2006
(51) Int. Cl.: A61K 31/46, A61P 11/00, C07D 451/10

(54) **PROCESS FOR PREPARING TIOTROPIUM SALTS, TIOTROPIUM SALTS AS SUCH AND PHARMACEUTICAL COMPOSITIONS THEREOF**
VERFAHREN ZUR HERSTELLUNG VON TIOTROPIUM SALZEN, TIOTROPIUM SALZE UND PHARMAZEUTISCHE MISCHUNGEN
PROCEDÉ DE FABRICATION DES SELS DE TIOTROPIUM, DES SELS DE TIOTROPIUM ET COMPOSITIONS PHARMACEUTIQUES

(30) Priority: 15.06.2005 EP 05105270
(43) Date of publication of application: 12.03.2008
(73) Proprietor: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: POP, Mihaela, NL-1060 NX, Amsterdam (NL); MULDER, HOUDAYER, Stéphanie, NL-3607 XD Maarssen (NL); SIEGER, Peter, 88441 Mittelbiberach (DE); PIEPER, Michael, P., 88400 Biberach (DE)
(74) Representative: Simon, Elke Anna Maria
(86) International application number: PCT/EP2006/062688
(87) International publication number: WO 2006/134021

(56) References cited:
- WO-A-02/30928
- WO-A-03/057694
- WO-A-2005/042526
- WO-A2-2004/064789
- US-A1- 2005 096 341
- BERGE S M ET AL: "PHARMACEUTICALS SALTS" JOURNAL OF PHARMACEUTICAL SCIENCES, AMERICAN PHARMACEUTICAL ASSOCIATION. WASHINGTON, US, vol. 66, no. 1, 1977, pages 1-19, XP000562636 ISSN: 0022-3549

## Description

The invention relates to a process for preparing new tiotropium salts.

### Background to the invention

Tiotropium bromide is known from European Patent Application EP 418 716 A1 and has the following chemical structure:

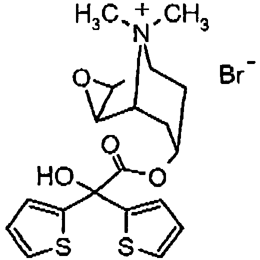

Tiotropium bromide is a highly effective anticholinergic with a long-lasting effect, which may be used to treat respiratory complaints, particularly COPD (chronic obstructive pulmonary disease) and asthma. By tiotropium is meant the free ammonium cation.

The aim of the present invention is to provide an alternative method of synthesis for preparing tiotropium salts which enables other tiotropium salts to be synthesised by a simple, non-aggressive method which is universally applicable.

The problem stated above is solved by the process according to the invention as described hereinafter.

The invention relates to a process for preparing new tiotropium salts of formula **1** 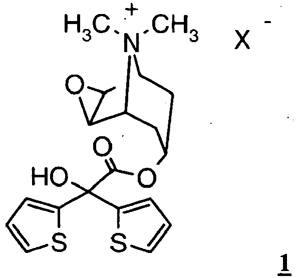 wherein
- X⁻: denotes an anion which is different from HCO₃⁻ (=bicarbonate)
characterised in that tiotropium bicarbonate of formula **2** 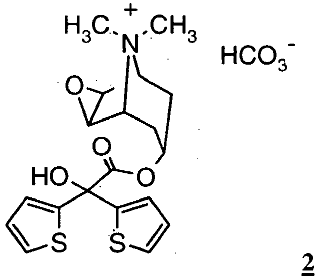 is reacted in a suitable solvent with an acid HX wherein X may have the meanings given above.

In a preferred embodiment the invention is directed to a process for the preparation of tiotropium salts of formula 1 wherein
- X⁻: denotes halide, HSO₄⁻, H₂PO₄⁻ or an anion selected from optionally substituted alkylsulphonate, optionally substituted alkenylsulphonate, optionally substituted alkinylsulphonate, optionally substituted cycloalkylsulphonate, optionally substituted alkylsulphate, optionally substituted alkenylsulphate, optionally substituted alkinylsulphate, optionally substituted cycloalkylsulphate, optionally substituted arylsulphonate, optionally substituted arylsulphate, optionally substituted heterocyclylsulphonate and optionally substituted heterocyclylsulphate;
or
- X⁻: denotes R-COO⁻, wherein R is hydrogen, COOH, COO-C₁-C₆-alkyl or a group selected from optionally substituted alkyl, optionally substituted alkenyl, optionally substituted alkinyl, optionally substituted cycloalkyl, optionally substituted aryl, and optionally substituted heterocyclyl.

In a yet another preferred embodiment the invention is directed to a process for the preparation of tiotropium salts of formula **1** wherein
- X⁻: denotes halide, HSO₄⁻, H₂PO₄⁻ or an anion selected from optionally substituted C₁-C₁₀-alkylsulphonate, optionally substituted C₂-C₁₀-alkenylsulphonate, optionally substituted C₂-C₁₀-alkinylsulphonate, optionally substituted C₃-C₈-cycloalkylsulphonate, optionally substituted C₁-C₁₀-alkylsulphate, optionally substituted C₂-C₁₀-alkenylsulphate, optionally substituted C₂-C₁₀-alkinylsulphate, optionally substituted C₃-C₈-cycloalkylsulphate, optionally substituted C₆-C₁₀-arylsulphonate, optionally substituted C₆-C₁₀-arylsulphate, optionally substituted heterocyclylsulphonate and optionally substituted heterocyclylsulphate;
or
- X⁻: denotes R-COO⁻, wherein R is hydrogen, COOH, COO-C₁-C₆-alkyl or a group selected from optionally substituted C₁-C₁₀-alkyl, optionally substituted C₂-C₁₀-alkenyl, optionally substituted C₂-C₁₀-alkinyl, optionally substituted C₃-C₈-cycloalkyl, optionally substituted C₆-C₁₀-aryl, and optionally substituted heterocyclyl.

In a yet another preferred embodiment the invention is directed to a process for the preparation of tiotropium salts of formula **1** wherein
- X⁻: denotes halide, HSO₄⁻, H₂PO₄⁻ or an anion selected from C₁-C₁₀-alkylsulphonate, C₂-C₁₀-alkenylsulphonate, C₂-C₁₀-alkinylsulphonate, C₃-C₈-cycloalkylsulphonate, C₁-C₁₀-alkylsulphate, C₂-C₁₀-alkenylsulphate, C₂-C₁₀-alkinylsulphate, C₃-C₈-cycloalkylsulphate, C₆-C₁₀-arylsulphonate, C₆-C₁₀-arylsulphate, heterocyclylsulphonate and heterocyclylsulphate, which is optionally substituted by one or more non-interfering groups;
or
- X⁻: denotes R-COO⁻, wherein R is hydrogen, COOH, COO-C₁-C₆-alkyl or a group selected from C₁-C₁₀-alkyl, C₂-C₁₀-alkenyl, C₂-C₁₀-alkinyl, C₃-C₈-cycloalkyl, C₆-C₁₀-aryl, and heterocyclyl, which is optionally substituted by one or more non-interfering groups.

In a yet another preferred embodiment the invention is directed to a process for the preparation of tiotropium salts of formula **1** wherein
- X⁻: denotes halide, HSO₄⁻, H₂PO₄⁻ or an anion selected from C₁-C₁₀-alkylsulphonate, C₂-C₁₀-alkenylsulphonate, C₂-C₁₀-alkinylsulphonate, C₃-C₈-cycloalkylsulphonate, C₁-C₁₀-alkylsulphate, C₂-C₁₀-alkenylsulphate, C₂-C₁₀-alkinylsulphate, C₃-C₈-cycloalkylsulphate, C₆-C₁₀-arylsulphonate, C₆-C₁₀-arylsulphate, heterocyclylsulphonate and heterocyclylsulphate, which is optionally substituted by one or more non-interfering groups which are preferably selected from halogen, OH, =O, CN, NO₂, NH₂, COOH, COO-C₁-C₆-alkyl, C₁-C₆-alkyl, C₁-C₆-haloalkyl, C₁-C₆-alkyloxy, C₃-C₈-cycloalkyl, C₆-C₁₀-aryl;
or
- X⁻: denotes R-COO⁻, wherein R is hydrogen, COOH, COO-C₁-C₆-alkyl or a group selected from C₁-C₁₀-alkyl, C₂-C₁₀-alkenyl C₂-C₁₀-alkinyl, C₃-C₈-cycloalkyl, C₆-C₁₀-aryl, and heterocyclyl, which is optionally substituted by one or more non-interfering groups which are preferably selected from halogen, OH, =O, CN, NO₂, NH₂, COOH, COO-C₁-C₆-alkyl, C₁-C₆-alkyl, C₁-C₆-haloalkyl, C₁-C₆-alkyloxy, C₃-C₈-cycloalkyl, C₆-C₁₀-aryl.

In a yet another preferred embodiment the invention is directed to a process for the preparation of tiotropium salts of formula **1** wherein
- X: denotes halide, HSO₄⁻, H₂PO₄⁻ or an anion selected from C₁-C₆-alkylsulphonate, C₂-C₆-alkenylsulphonate, C₂-C₆-alkinylsulphonate, C₃-C₆-cycloalkylsulphonate, C₁-C₆-alkylsulphate, C₂-C₆-alkenylsulphate, C₂-C₆-alkinylsulphate, C₃-C₆-cycloalkylsulphate, C₆-C₁₀-arylsulphonate, C₆-C₁₀-arylsulphate, heterocyclylsulphonate and heterocyclylsulphate, which is optionally substituted by one or more non-interfering groups being preferably selected from halogen, OH, =O, CN, NO₂, NH₂, COOH, COO-C₁-C₄-alkyl, C₁-C₄-alkyl, C₁-C₄-haloalkyl, C₁-C₄-alkyloxy, C₃-C₆-cycloalkyl, C₆-C₁₀-aryl;
or
- X⁻: denotes R-COO⁻, wherein R is hydrogen, COOH, COO-C₁-C₆-alkyl or a group selected from C₁-C₆-alkyl, C₂-C₆-alkenyl, C₂-C₆-alkinyl, C₃-C₆-cycloalkyl, C₆-C₁₀-aryl, and heterocyclyl, which is optionally substituted by one or more non-interfering groups being preferably selected from halogen, OH, =O, CN, NO₂, NH₂, COOH, COO-C₁-C₆-alkyl, C₁-C₆-alkyl, C₁-C₆-haloalkyl, C₁-C₆-alkyloxy, C₃-C₈-cycloalkyl, C₆-C₁₀-aryl.

In a yet another preferred embodiment the invention is directed to a process for the preparation of tiotropium salts of formula **1** wherein
- X⁻: denotes chloride, bromide, iodide, HSO₄⁻, H₂PO₄⁻ or an anion selected from C₁-C₆-alkylsulphonate, C₁-C₆-alkylsulphate, phenylsulphonate, and naphthylsulphonate, which is optionally substituted by one or more non-interfering groups being preferably selected from chlorine, bromine, fluorine, OH, =O, CN, NO₂, NH₂, COOH, COO-C₁-C₄-alkyl, C₁-C₄-alkyl, C₁-C₄-fluoroalkyl, C₁-C₄-alkyloxy, C₃-C₆-cycloalkyl, phenyl, and naphthyl;
or
- X⁻: denotes R-COO⁻, wherein R is hydrogen, COOH, or a group selected from C₁-C₆-alkyl, C₂-C₆-alkenyl, C₂-C₆-alkinyl, C₃-C₆-cycloalkyl, phenyl, naphthyl, and heterocyclyl, which is optionally substituted by one or more non-interfering groups being preferably selected from chlorine, bromine, fluorine, OH, =O, CN, NO₂, NH₂, COOH, COO-C₁-C₄-alkyl, C₁-C₄-alkyl, C₁-C₄-fluoroalkyl, C₁-C₄-alkyloxy, C₃-C₆-cycloalkyl, phenyl, naphthyl.

In a yet another preferred embodiment the invention is directed to a process for the preparation of tiotropium salts of formula **1** wherein
- X⁻: denotes chloride, bromide, iodide, HSO₄⁻, H₂PO₄⁻ or an anion selected from methanesulphonate, benzenesulphonate, toluenesulphonate, trifluoromethansulphonate,
or
- X⁻: denotes R-COO⁻, wherein R is hydrogen, COOH, methyl, ethyl, propyl, -CH₂-OH, -CH₂-CH₂-OH, -CH₂-CH₂-CH₂-OH, -CH₂-CH₂-CH₂-CH₂-OH, -C(OH)H-CH₃, -C(OH)H-CH₂-CH₃, -C(OH)H-CH₂-CH₂-CH₃, -C(OH)H-CH₂-CH₂-CH₂-CH₃, -CH₂-COOH, -CH₂-CH₂-COOH, -CH₂-CH₂-CH₂-COOH, -CH₂-CH₂-CH₂-CH₂-COOH, -C(OH)H-COOH, -C(OH)H-CH₂-COOH, -C(OH)H-CH₂-CH₂-COOH, -C(OH)H-CH₂-CH₂-CH₂-COOH, -C(NH₂)H-COOH, -C(NH₂)H-CH₂-COOH, -C(NH₂)H-CH₂-CH₂-COOH, -C(NH₂)H-CH₂-CH₂-CH₂-COOH, -C(OH)H-C(OH)H-COOH, -C(OH)H-C(OH)H-CH₂-COOH, -C(OH)H-C(OH)H-CH₂-CH₂-COOH, -C(OH)H-C(OH)H-C(OH)H-COOH, -C(OH)H-C(OH)H-C(OH)H-C(OH)H-COOH, -CH=CH2, -CH=CH-CH₃, -CH=CH-COOH,
or
- X⁻: denotes R-COO⁻, wherein R is a group selected from cyclopentyl and cyclohexyl, being optionally substituted by one or more groups selected from among =O, OH, COOH, methyl, ethyl and fluorine,
or
- X⁻: denotes R-COO⁻, wherein R is a group selected from phenyl, benzyl, phenylethyl, and naphthyl, being optionally substituted by one or more groups selected from among =O, OH, COOH, methyl, ethyl, fluorine, chlorine, CF₃, phenyl, benzyl, naphthyl, napthylmethyl, and naphthylmethyl which is substituted by OH and/or COOH.

In a yet another preferred embodiment the invention is directed to a process for the preparation of tiotropium salts of formula **1** wherein
- X⁻: denotes chloride, iodide, HSO₄⁻, H₂PO₄⁻, benzenesulphonate, toluenesulphonate, methanesulphonate, trifluoromethanesulphonate, xinafoate, malate, aspartate, succinate, camphorate, acetate, propionate, fumarate, isobutyrate, malonate, oxalate, salicylate, tartrate, ethandisulfonate, gluconate, glutarate, lactate, citrate, maleinate, saccharinate and pamoate.

In case X⁻ denotes an anion which possesses a second acidic hydrogen the process according to the invention may also lead to compounds according to formula **1a** 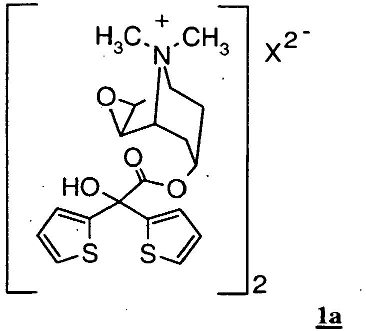 wherein X²⁻ is a di-anion selected from those anions X⁻ mentioned hereinbefore that are able to form a di-anion by donation of another H⁺. For a person of ordinary skill in the art it is clear which of the aforementioned groups X are capable to form such a di-anion. As an example are to be mentioned for instance HSO₄⁻, H₂PO₄⁻, and all those groups X that possess a second COOH - group as specified hereinbefore.

The invention relates to a process for the preparation of compounds of formula **1** optionally in the form of the individual optical isomers, mixtures of the individual enantiomers or racemates, and optionally to the hydrates and/or solvates thereof.

The alkyl groups meant here (including those which are components of other groups) are branched and unbranched alkyl groups having 1 to 10 carbon atoms, preferably 1 to 4 carbon atoms, such as: methyl, ethyl, n-propyl, iso-propyl, butyl, iso-butyl, sec.-butyl, tert.-butyl, pentyl, iso-pentyl, hexyl, heptyl and octyl.

Unless otherwise specified, substituted alkyl groups (including those which are components of other groups) may, for example, carry one or more of the following substituents: halogen, hydroxy, mercapto, C₁₋₆-alkyloxy, amino, alkylamino, dialkylamino, cyano, nitro, =O, -CHO, -COOH, -COO-C₁₋₆-alkyl, -S-C₁₋₆-alkyl.

Alkenyl groups (including those which are components of other groups) are the branched and unbranched alkenyl groups with 2 to 10 carbon atoms, preferably 2 to 3 carbon atoms, provided that they have at least one double bond, e.g. the alkyl groups mentioned above provided that they have at least one double bond, such as for example vinyl (provided that no unstable enamines or enolethers are formed), propenyl, iso-propenyl, butenyl, pentenyl and hexenyl.

Unless otherwise specified, substituted alkenyl groups, (including those which are components of other groups), may for example carry one or more of the following substituents: halogen, hydroxy, mercapto, C₁₋₆-alkyloxy, amino, alkylamino, dialkylamino, cyano, nitro, =O, -CHO, -COOH, -COO-C₁₋₆-alkyl, -S-C₁₋₆-alkyl.

The term alkinyl groups (including those which are components of other groups) refers to alkinyl groups having 2 to 10 carbon atoms provided that they have at least one triple bond, e.g. ethinyl, propargyl, butinyl, pentinyl and hexinyl.

Unless otherwise specified, substituted alkinyl groups, (including those which are components of other groups), may for example carry one or more of the following substituents: halogen, hydroxy, mercapto, C₁₋₆-alkyloxy, amino, alkylamino, dialkylamino, cyano, nitro, =O, -CHO, -COOH, -COO-C₁₋₆-alkyl, -S-C₁₋₆-alkyl.

Examples of cycloalkyl groups having 3 to 8 carbon atoms include cyclopropyl, cyclobutyl, cyclopentyl, cyclohexyl, cycloheptenyl and cyclooctyl which may also be substituted by branched or unbranched C₁₋₄-alkyl, hydroxy and/or halogen or as hereinbefore defined. The term halogen generally refers to fluorine, chlorine, bromine or iodine.

The word aryl denotes an aromatic ring system having 6 to 10 carbon atoms which, unless otherwise specified, may carry one or more of the following substituents, for example: C₁₋₆-alkyl, C₁₋₆-alkyloxy, halogen, hydroxy, mercapto, amino, alkylamino, dialkylamino, CF₃, cyano, nitro, -CHO, -COOH, -COO-C₁₋₆-alkyl, -S-C₁₋₆-alkyl. The preferred aryl group is phenyl.

The term heterocyclyl denotes cyclic groups possessing at least one heteroatom which may contain nitrogen, oxygen or sulphur as heteroatoms. Examples include furan, tetrahydrofuran, tetrahydrofuranone, γ-butyrolactone, α-pyran, γ-pyran, dioxolane, tetrahydropyran, dioxane, thiophene, dihydrothiophene, thiolane, dithiolane, pyrrole, pyrroline, pyrrolidine, pyrazole, pyrazoline, imidazole, imidazoline, imidazolidine, triazole, tetrazole, pyridine, piperidine, pyridazine, pyrimidine, pyrazine, piperazine, triazine, tetrazine, morpholine, thiomorpholine, oxazole, isoxazole, oxazine, thiazole, isothiazole, thiadiazole, oxadiazole and pyrazolidine, preferably morpholine, piperazine and piperidine, wherein the above-mentioned heterocycles may also be substituted by benzyl or C₁₋₄-alkyl, preferably methyl.

"=O" means an oxygen atom linked by a double bond.

A non-interfering group is to be understood as a group which leaves the activity of the compounds **1** in the intended use qualitatively intact. The intended use of the compounds of formula **1** is specified in more detail below. Examples of non-interfering groups within the scope of the instant invention are selected from halogen, OH, =O, CN, NO₂, NH₂, COOH, COO-C₁-C₆-alkyl, C₁-C₆-alkyl, C₁-C₆-haloalkyl, C₁-C₆-alkyloxy, C₃-C₈-cycloalkyl, C₆-C₁₀-aryl. Further examples of non-interfering groups include phenyl-C₁-C₆-alkylen or napthyl-C₁-C₆-alkylene which are optionally substituted by one or more groups selected from methyl, ethyl, OH, COOH, COO-C₁-C₄-alkyl and CF₃, preferably OH or COOH.

In the process according to the invention the tiotropium bicarbonate is dissolved or suspended in a suitable solvent. Suitable solvents include water or organic solvents, preferably water or polar organic solvents that are suitable to dissolve tiotropium bicarbonate **2**. Preferred solvents are protic solvents such as alcohols (for example methanol, ethanol, isopropanol) and water, preferably water of pH 2-6 as well as polar organic solvents selected from the group consisting of alcohols such as for example ethyleneglycol and diethyleneglycol, amides such as for example dimethylformamide and N-methyl-pyrrolidinone, ethers such as for example tetrahydrofuran, dioxane, dimethylether and nitriles such as for example acetonitrile. It is particularly preferable to use water, methanol, ethanol, isopropanol, ethyleneglycol, diethyleneglycol, dimethylformamide, N-methyl-pyrrolidinone, tetrahydrofuran, dioxane, dimethylether or acetonitrile as solvent, while water, particularly aqueous solutions with a pH of about 2-6 are particularly preferred according to the invention. The solution of tiotropium bicarbonate **2** in one of the aforementioned solvents is treated with the acid HX, wherein X may have the meanings specified herein. Preferably the tiotropium bicarbonate **2** solution is treated with HX at a pH below 5, preferably below 4, more preferably in a range of pH 2-3. This pH is adjusted by the acid HX.

Progress of the transformation can be detected for instance via ¹H-NMR or other methods, well known to the person of ordinary skill in the art.

When the transformation is complete, the solution is cooled down to a temperature below 15°C, preferably below 10°C, more preferably to 3-5°C. Usually, the salts of formula **1** crystallize from the reaction solution.

In case crystallization does not occur, the solvent is removed and the remaining residue, if not crystalline recrystallized from alcohol, preferably ethanol.

In a particular preferred embodiment according to the invention, the process is conducted using a suitable ion exchange resin. Preferred ion exchange resins are basic anion exchange resins based on polystyrene, optionally cross linked with divinyl benzene or polyethylenglycol. Of particular interest are ion exchange resins based on styrene-divenylbenzen basis. Within the scope of the invention the term ion exchange resin is occasionally also abbreviated by the term IER.

In a particular preferred embodiment of the invention an ion exchange resin as specified hereinbefore is already used for the preparation of the tiotropium bicarbonate **2** starting material.

This preparation of the bicarbonate **2** is preferably conducted as follows. An ion exchange resin (IER) suitably charged with bicarbonate-ions is treated with a solution of a tiotropium salt already known in the art in a suitable solvent. Preferably tiotropium bromide is used as the starting material, more preferably tiotropium bromide monohydrate as known from WO 02/30928 is used. Other tiotropium salts and salt forms that are suitable as staring materials for the preparation of the bicarbonate **2** are disclosed for instance in EP 0418716, WO 03/000265, WO 05/042526, WO 05/042528, and WO 05/042527. Suitable solvents include water or organic solvents, preferably water or polar organic solvents that are suitable to dissolve tiotropium bicarbonate **2**. Preferred solvents are protic solvents such as alcohols (for example methanol, ethanol, isopropanol) and water, preferably water of pH 2-6 as well as polar organic solvents selected from the group consisting of alcohols such as for example ethyleneglycol and diethyleneglycol, amides such as for example dimethylformamide and N-methyl-pyrrolidinone, ethers such as for example tetrahydrofuran, dioxane, dimethylether and nitriles such as for example acetonitrile. It is particularly preferable to use water, methanol, ethanol, isopropanol, ethyleneglycol, diethyleneglycol, dimethylformamide, N-methyl-pyrrolidinone, tetrahydrofuran, dioxane, dimethylether or acetonitrile as solvent, while water, particularly aqueous solutions with a pH of about 2-6 are particularly preferred according to the invention.

The tiotropium bicarbonate **2** can be obtained from the IER in form of a solution by washing thereof with one of the solvents mentioned hereinbefore. Preferred solvent is water. The bicarbonate **2** containing solution can be detected in the washing procedure via a UV reader working at 240 nm and is preferably stored in form of this solution without further isolation of pure **2**. **2** could be isolated via removal of the solvent according to known methods (i.e. evaporation of solvent or freeze drying). However, in these cases at least partial decomposition of the bicarbonate **2** may occur.

A yet another preferred embodiment according to the invention relates to the preparation of tiotropium bicarbonate **2** as specified hereinbefore.

The tiotropium bicarbonate **2** is an extremely valuable intermediate for the easy preparation of tiotropium salts **1**. Consequently, in another embodiment, the instant invention relates to tiotropium bicarbonate **2** 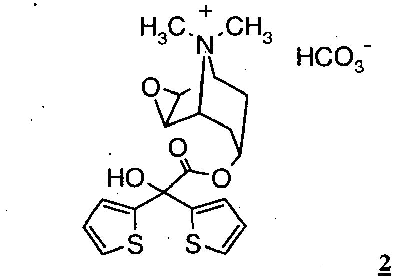 per se.

In another embodiment, the invention relates to solutions containing **2** dissolved or suspended, preferably dissolved in a solvent. In a yet another preferred embodiment the invention relates to solutions of **2** in a solvent such as alcohols (for example methanol, ethanol, isopropanol) and water, preferably water of pH 2-6 as well as solutions in polar organic solvents selected from the group consisting of alcohols such as for example ethyleneglycol and diethyleneglycol, amides such as for example dimethylformamide and N-methyl-pyrrolidinone, ethers such as for example tetrahydrofuran, dioxane, dimethylether and nitriles such as for example acetonitrile. Particularly preferred are solutions of **2** in a solvent selected from among water, methanol, ethanol, isopropanol, ethyleneglycol, diethyleneglycol, dimethylformamide, N-methyl-pyrrolidinone, tetrahydrofuran, dioxane, dimethylether or acetonitrile, while solutions in water, particularly with a pH of about 2-6 are particularly preferred according to the invention.

Another embodiment according to the invention is directed to the use of bicarbonate **2** as a starting material for the preparation of tiotropium salts **1**.

The Examples that follow serve to illustrate the present invention still further.

### Examples of synthesis according to the invention and characterization of products

### Methods:

### Single Crystal X-ray diffraction

Suitable single crystal selected after the crystallization experiments, were glued to a glass fibre, which was mounted on an X-ray diffraction goniometer. X-ray diffraction data was collected for these crystals at a temperature of 233 K using a *Kappa*CCD system and Mo*Kα* radiation generated by a FR590 X-ray generator (Bruker Nonius, Delft, The Netherlands).

Unit-cell parameters and crystal structures were determined and refined using the software package maXus (Mackay *et al.*, 1997). From the crystal structure the theoretical X-ray powder diffraction pattern were calculated using PowderCell for Windows version 2.3 (Kraus *et al.,* 1999).

### X-ray powder diffraction

X-Ray powder diffraction patterns were obtained using Avantium's T2 high throughput XRPD set-up. The plates were mounted on a Bruker GADDS diffractometer that is equipped with a Hi-Star area detector. The XRPD platform is calibrated using Silver Behenate for the long d-spacings and Corundum for the short d-spacings.

The data collection was carried out at room temperature using monochromatic CuK_{α} radiation in the region of 2θ between 1.5 and 41.5 °. The diffraction pattern of each well was collected in two 2theta ranges (1.5 ≤ 2θ ≤ 19.5 ° for the 1st frame, and 21.5 ≤ 2θ ≤ 41.5 ° for the second frame) with an exposure time between 90 and 180 s for each frame. No background subtraction or curve smoothing was applied to the XRPD patterns.

### Melting points - Differential Scanning Calorimetry

Melting properties were obtained from differential scanning calorimetry (DSC) thermograms, recorded with a DSC822e (Mettler-Toledo GmbH, Switzerland). The DSC822e is calibrated for temperature and enthalpy with a small piece of indium (m.p. = 156.6°C; ΔHf = 28.45 J.g⁻¹). Samples are sealed in standard 40 *µ*l aluminum pans and heated in the DSC from 25 to 300°C with a heating rate of 10 and 20°C min⁻¹. Dry N₂ gas is used to purge the DSC equipment during measurement at a flow rate of 50 ml min⁻¹.

### Conditioning of ion exchange resin:

For the preparation of the different salt forms of tiotropium an anionic ion exchange resin (= IER) was used. Prior to all preparations the following way of conditioning was applied to the IER:

A column (length: 1.5 m; diameter: 4 cm) was charged with 3 x 100 g of Ion Exchange Resin (IER) (Dowex 1x8-200 Cl, Aldrich 21,742-5, Lot: 01428JB, 1.5 eq/ml). The column was rinsed with 4 l of a saturated aqueous NaHCO₃ solution (Aldrich 34,094-4, Lot: 505422-203 in demineralised water) with a flow of ca. 15 ml/min. The completion of the exchange was checked by taking a 2 ml sample of the elute that was acidified with a 1 M HNO3 (aq) solution and adding 2 drops of a 0.5 M AgNO₃ (aq) solution to the stirred solution. A precipitate indicates the presence of Cl⁻ ions while a clear solution indicates completion of the exchange. Next, the column was rinsed with demineralised water until the pH was essentially neutral (pH = 6-7) and identical to demineralised water.

Approximately 9 l of water was required to achieve this (flow 15 ml/min). The IER was transferred to an Erlenmeyer flask and was kept as slurry.

### Preparation of Tiotropium bicarbonate 2:

A glass column (ca. 110 ml) was filled with the dry ion exchange resin and afterwards filled with deionized water. The column was loaded with bicarbonate using a saturated NaHCO3-solution (ca. 90 g NaHCO3/l). About 4,5 l of this saturated NaHCO3-solution were passed through the column with a flow rate of approx. 10 ml/min. Afterwards the column was washed with 6 l of deionized water (flow rate: 20 ml/min) to get rid of excess of NaHCO3. 50 ml of a tiotropiumbromide solution (conc. 10 mg/ml) are slowly (flow rate: 2 ml/min) passed on the column. Afterwards the column is washed with deionized water using a flow rate of 5 ml/min. Eluation of tiotropiumbicarbonate **2** from the column was detected using a UV-reader working at 240 nm. The fraction containing tiotropiumbicarbonate was collected (approx. 150 ml), the concentration was approx. 3 mg/ml, the pH was around 6.5.

The tiotropium bicarbonate **2** is preferably stored in form of this solution. In the alternative **2** is freshly prepared in advance to every salt form preparation. This procedure is usually applied in the following examples.

### Example 1: crystalline anhydrous tiotropium benzenesulphonate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of benzenesulfonic acid in 10 ml of water. The resulting solution was stored at 4°C in the refrigerator overnight. The next day crystals of the besylate of tiotropium were formed. These were filtered, washed with cold demineralised water and dried under vacuum. Melting point: 237+3°C (DSC); the crystal structure of this salt was solved by single crystal X-ray diffraction analysis (see table 1).

**Table 1:**

| | |
|---|---|
| Empirical formula | C₁₉H₂₂NO₄S₂⁺ • C₆H₅SO₃⁻ |
| Formula weight | 549.66 |
| T[K] | 293(2) |
| λ[Å] | 0.71073 |
| Crystal system | Orthorhombic |
| Space group | Pbca |

| Unit cell dimensions | |
|---|---|
| a [Å] | 10.6460(7) |
| b [Å] | 12.8410(10) |
| c [Å] | 36.605(3) |
| V [Å³] | 5004.1(6) |
| Z | 8 |
| Dₘ [g/cm³] | 1.459 |
| F(000) | 2304 |
| Crystal size [mm³] | 0.7 x 0.4 x 0.1 |
| θ range | 3 to 27°. |
| Reflections collected | 10619 |
| Independent reflections | 5640 [Rᵢₙₜ = 0.0284] |
| Data / restraints / parameters | 5640 / 0 / 458 |
| S | 1.024 |
| R [I>2σ(I)] | R1 = 0.0557, wR2 = 0.1398 |
| R indices (all data) | R1 = 0.0766, wR2 = 0.1523 |
| Extinction coefficient | 0.0023(7) |

### Example 2: crystalline anhydrous tiotropium trifluoromethanesulphonate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of trifluormethanesulfonic acid in 10 ml of water. The resulting solution was stored at 4°C in the refrigerator overnight. The next day crystals of the triflate of tiotropium were formed. These were filtered, washed with cold demineralised water and dried under vacuum. Melting point: 188+3°C (DSC); the crystal structure of this salt was solved by single crystal X-ray diffraction analysis (see table 2).

**Table 2:**

| | |
|---|---|
| Empirical formula | C₁₉H₂₂NO₄S₂⁺ • CF₃SO₃⁻ |
| Fw | 541.57 |
| T [K] | 293(2) K |
| λ[Å] | 0.71073 Å |
| Crystal system | Monoclinic |
| Space group | P 2₁/c |

| Unit cell dimensions | |
|---|---|
| a [Å] | 12.4500(7) |
| b [Å] | 13.1090(9) |
| c [Å] | 17.9840(14) |
| β [°] | 129.176(2) |
| V [Å³] | 2275.3(3) |
| Z | 4 |
| Dₘ [g/cm³] | 1.581 |
| F(000) | 1120 |
| Crystal size [mm³] | 0.6 x 0.5 x 0.4 |
| θ range[°] | 2 → 27.5. |
| Reflections collected | 15539 |
| Independent reflections | 5198 [Rᵢₙₜ = 0.0353] |
| Data / restraints / parameters | 5198 / 0 / 411 |
| S | 1.021 |
| R [I>2σ(I)] | R1 = 0.0466, wR2 = 0.1143 |
| R indices (all data) | R1 = 0.0641, wR2 = 0.1269 |
| Extinction coefficient | 0.028(2) |

### Example 3: crystalline tiotropium salicylate monohydrate (form I)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of salicylic acid in 10 ml of water. From the resulting solution water was evaporated under vacuum over a water bath which was kept below 35 °C. The obtained solid was further dried under vacuum yielding a hydrated form of the salicylate of tiotropium. The crystal structure of this salt was solved by single crystal X-ray diffraction analysis (see table 3).

**Table 3:**

| | |
|---|---|
| Empirical formula | C₁₉H₂₂NO₄S₂⁺ • C₇H₅O₃⁻ • H₂O |
| Fw | 547.62 |
| T [K] | 293(2) K |
| λ[Å] | 0.71073 Å |
| Crystal system | Triclinic |
| Space group | P -1 |

| Unit cell dimensions | |
|---|---|
| a [Å] | 10.8380(3) |
| b [Å] | 10.8610(3) |
| c [Å] | 12.2310(4) |
| α[°] | 76.199(2) |
| β[°] | 71.878(2) |
| γ[°] | 74.220(2) |
| V [Å³] | 1297.95(7) |
| Z | 2 |
| Dₘ [g/cm³] | 1.401 |
| F(000) | 576 |
| Crystal size [mm³] | 0.6 x 0.4 x 0.2 |
| θ range[°] | 3 to 27.5. |
| Reflections collected | 6946 |
| Independent reflections | 5568 [Rᵢₙₜ = 0.0286] |
| Data / restraints / parameters | 5568 / 0 / 450 |
| S | 1.023 |
| R [I>2σ(I)] | R1 = 0.0507, wR2 = 0.1208 |
| R indices (all data) | R1 = 0.0744, wR2 = 0.1365 |

### Example 4: crystalline anhydrous tiotropium salicylate (form II)

1.00 g of tiotropium bromide monohydrate (according to WO 02/30928) was dissolved in in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of salicylic acid in 10 ml of water. From the resulting solution water was evaporated under vacuum over a water bath which was kept below 35 °C. The obtained solid was further dried under vacuum yielding crystalline material of the salicylate of tiotropium.

Approx. 600 mg of the crystalline tiotropium salicylate are dissolved in 4 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a mixture of 1-butanol/methanol = 50:50 was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 25 °C. At this temperature the plate remained for a hold time of 24 h.

The plates are opened afterwards the solid is obtained by evaporation of the solvent at room temperature in a vacuum chamber (13 kPa). Melting point: 195+3°C (DSC); table 4a summarizes the X-ray powder reflections obtained for this form.

**Table 4a:**

| **2 Θ [°]** | **d [Å]** | **I/Iₒ [%]** |
|---|---|---|
| 6.54 | 13.50 | 56 |
| 9.42 | 9.38 | 11 |
| 10.54 | 8.38 | 11 |
| 12.22 | 7.23 | 79 |
| 12.86 | 6.88 | 7 |
| 13.86 | 6.38 | 16 |
| 16.22 | 5.46 | 100 |
| 16.82 | 5.26 | 42 |
| 18.38 | 4.82 | 23 |
| 19.54 | 4.54 | 31 |
| 20.62 | 4.30 | 97 |
| 21.98 | 4.04 | 23 |
| 22.98 | 3.87 | 9 |
| 23.42 | 3.79 | 14 |
| 24.38 | 3.65 | 8 |
| 25.06 | 3.55 | 18 |
| 26.06 | 3.42 | 27 |
| 27.38 | 3.25 | 21 |
| 28.10 | 3.18 | 6 |
| 29.34 | 3.06 | 10 |
| 29.82 | 3.02 | 7 |

The crystal structure of this salt was also solved by single crystal X-ray diffraction analysis (see table 4b).

**Table 4b:**

| | |
|---|---|
| Empirical formula | C₁₉H₂₂NO₄S₂⁺ • C₇H₅O₃⁻ |
| Formula weight | 529.61 |
| Temperature | 293(2) |
| Wavelength | 0.71073 |
| Crystal system | Monoclinic |
| Space group | C 2/c |

| Unit cell dimensions | |
|---|---|
| a [Å] | 13.273(2) |
| b [Å] | 13.865(2) |
| c [Å] | 28.042(4) |
| β[°] | 101.98(2) |
| V [Å³] | 5048.1(10) |
| Z | 8 |
| Density (calculated) | 1.394 |
| F(000) | 2224 |
| Crystal size | 0.3 x 0.2 x 0.2 |
| Theta range for data collection | 1.5 → 25 |
| Reflections collected | 7741 |
| Independent reflections | 4037 [Rᵢₙₜ = 0.0526] |
| Data / restraints / parameters | 4037 / 0 / 335 |
| Goodness-of-fit on F² | 1.090 |
| Final R indices [I>2sigma(I)] | R1 = 0.0842, wR2 = 0.2068 |
| R indices (all data) | R1 = 0.1453, wR2 = 0.2482 |
| Extinction coefficient | 0.0046(8) |

### Example 5: crystalline anhydrous tiotropium salicylate (form III)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of salicylic acid in 10 ml of water. From the resulting solution water was evaporated under vacuum over a water bath which was kept below 35 °C. The obtained solid was further dried under vacuum yielding crystalline material of the salicylate of tiotropium.

Approx. 600 mg of the crystalline tiotropium salicylate are dissolved in 4 ml of a mixture of acetone/water = 80:20. In two steps 75 *µ*l(40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of N,N-dimethyl-acetamide (= DMA) was added to this vial. The whole 96 well plate is seated afterwards and heated up with a heating rate of 5 °C/min to 50 °C at which the plate stays for an additional 30 minutes. Afterwards the plate is cooled with a cooling rate of 5°C/h to a final temperature of 5 °C. At this temperature the plate remained for a hold time of 24 h. The plates are opened afterwards the solid is obtained by evaporation of the solvent at room temperature in a vacuum chamber (13 kPa). Table 5 summarizes the X-ray powder reflections obtained for this form.

**Table 5:**

| **2 Θ [°]** | **d [Å]** | **I/Iₒ [%]** |
|---|---|---|
| 8.18 | 10.80 | 51 |
| 10.86 | 8.14 | 16 |
| 11.22 | 7.88 | 22 |
| 12.98 | 6.81 | 15 |
| 13.62 | 6.49 | 52 |
| 14.18 | 6.24 | 15 |
| 15.74 | 5.62 | 17 |
| 16.34 | 5.42 | 83 |
| 17.70 | 5.00 | 37 |
| 18.94 | 4.68 | 34 |
| 20.74 | 4.28 | 58 |
| 21.82 | 4.07 | 15 |
| 22.58 | 3.93 | 100 |
| 23.42 | 3.79 | 27 |
| 23.98 | 3.71 | 18 |
| 24.98 | 3.56 | 33 |
| 26.02 | 3.42 | 16 |
| 27.78 | 3.21 | 7 |
| 28.66 | 3.11 | 12 |

### Example 6: crystalline tiotropium salicylate monohydrate (form IV)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of salicylic acid in 10 ml of water. From the resulting solution water was evaporated under vacuum over a water bath which was kept below 35 °C. The obtained solid was further dried under vacuum yielding crystalline material of the salicylate of tiotropium. Approx. 600 mg of the crystalline tiotropium salicylate are dissolved in 4. ml of a mixture of acetone/water = 80:20. The solvent was gently evaporated at room temperature in a vacuum chamber (13 kPa). The crystalline material obtained was tiotropium salicylate, form IV which is another hydrated form (monohydrate II) of tiotropium salicylate. Melting point of 193±3 °C (DSC); table 6 summarizes the X-ray powder reflections obtained for this form.

**Table 6:**

| **2 Θ [°]** | **d [Å]** | **I/Iₒ [%]** |
|---|---|---|
| 7.38 | 11.98 | 22 |
| 8.74 | 10.12 | 84 |
| 10.86 | 8.15 | 8 |
| 12.06 | 7.34 | 20 |
| 13.14 | 6.74 | 5 |
| 13.66 | 6.48 | 38 |
| 14.14 | 6.26 | 13 |
| 14.58 | 6.07 | 27 |
| 15.58 | 5.69 | 6 |
| 16.82 | 5.27 | 17 |
| 17.54 | 5.06 | 83 |
| 18.06 | 4.91 | 53 |
| 19.02 | 4.67 | 23 |
| 20.02 | 4.43 | 11 |
| 20.66 | 4.30 | 3 |
| 21.86 | 4.07 | 100 |
| 22.70 | 3.92 | 47 |
| 23.18 | 3.84 | 22 |
| 23.58 | 3.77 | 31 |
| 24.18 | 3.68 | 11 |
| 24.42 | 3.64 | 10 |
| 25.26 | 3.53 | 32 |
| 26.66 | 3.34 | 7 |
| 27.78 | 3.21 | 15 |
| 28.82 | 3.10 | 4 |
| 29.18 | 3.06 | 11 |
| 29.62 | 3.02 | 5 |

### Example 7: crystalline tiotropium hydrogenesulphate monohydrate (form 1)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of sulphuric acid in 10 ml of water. The resulting solution was stored at 4°C in the refrigerator overnight. The next day crystals of a hydrated form of the hydrogenesulphate of tiotropium were formed. These were filtered, washed with cold demineralised water and dried under vacuum. The crystal structure of this salt was solved by single crystal X-ray diffraction analysis (see table 7).

**Table 7:**

| | |
|---|---|
| Empirical formula | C₁₉H₂₂NO₄S₂⁺ • HSO₄ • H₂O |
| Fw | 507.58 |
| T [K] | 293(2)K |
| λ[Å] | 0.71073 |
| Crystal system | Triclinic |
| Space group | P -1 |

| Unit cell dimensions | |
|---|---|
| a [Å] | 9.3750(2) |
| b [Å] | 11.6470(2) |
| c [Å] | 20.5450(5) |
| α[°] | 91.6260(9) |
| β[°] | 95.7210(9) |
| γ[°] | 91.8520(12) |
| V [Å³] | 2229.85(8) |
| Z | 4 |
| Dₘ [g/cm³] | 1.509 |
| F(000) | 1060 |
| Crystal size [mm³] | 0.4 x 0.4 x 0.1 |
| θ range[°] | 2 to 27.5. |
| Reflections collected | 15298 |
| Independent reflections | 10156 [Rᵢₙₜ = 0.0317] |
| Data / restraints / parameters | 10156 / 0 / 624 |
| S | 1.016 |
| R [I>2σ(I)] | R1 = 0.0699, wR2 = 0.1744 |
| R indices (all data) | R1 = 0.1278, wR2 = 0.2086 |
| Extinction coefficient | 0.0010(9) |

### Example 8: crystalline anhydrous tiotropium hydrogenesulphate (form II)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of sulphuric acid in 10 ml of water. The resulting solution was stored at 4°C in the refrigerator overnight. The next day crystals of a hydrated form of the hydrogenesulphate of tiotropium were formed. These were filtered, washed with cold demineralised water and dried under vacuum.

Approx. 900 mg of the crystalline tiotropium hydrogenesulphate are dissolved in 2 ml of water. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a mixture of 1-butanol/methanol = 50:50 was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 25 °C. At this temperature the plate remained for a hold time of 24 h. The plates are opened afterwards the solid is obtained by evaporation of the solvent at room temperature in a vacuum chamber (13 kPa). Melting point: 188±3°C (DSC); table 8a summarizes the X-ray powder reflections obtained for this form.

**Table 8a:**

| **2 Θ [°]** | **d [Å]** | **I/Iₒ [%]** |
|---|---|---|
| 5.38 | 16.41 | 21 |
| 6.18 | 14.28 | 10 |
| 7.94 | 11.12 | 14 |
| 11.46 | 7.71 | 40 |
| 12.30 | 7.19 | 27 |
| 13.62 | 6.49 | 21 |
| 15.62 | 5.67 | 79 |
| 16.50 | 5.37 | 38 |
| 17.06 | 5.21 | 17 |
| 17.66 | 5.02 | 11 |
| 18.50 | 4.79 | 66 |
| 19.06 | 4.65 | 54 |
| 19.62 | 4.52 | 44 |
| 20.30 | 4.37 | 36 |
| 20.78 | 4.27 | 39 |
| 21.62 | 4.11 | 46 |
| 22.46 | 3.95 | 100 |
| 23.06 | 3.85 | 33 |
| 24.26 | 3.66 | 17 |
| 24.94 | 3.57 | 26 |
| 25.50 | 3.49 | 19 |
| 26.10 | 3.41 | 17 |
| 27.30 | 3.26 | 31 |
| 28.34 | 3.15 | 13 |
| 29.18 | 3.06 | 20 |

The crystal structure of this salt was also solved by single crystal X-ray diffraction analysis (see table 8b).

**Table 8b:**

| | |
|---|---|
| Empirical formula | C₁₉H₂₂NO₄S₂•HSO₄⁻ |
| Formula weight | 489.56 |
| Temperature | 293(2) K |
| Wavelength | 0.71073 Å |
| Crystal system | Monoclinic |
| Space group | P 2₁/c |

| Unit cell dimensions | |
|---|---|
| a [Å] | 8.0390(2) Å |
| b [Å] | 15.989(1) Å |
| c [Å] | 33.190(2) Å |
| β[°] | 90.265(2) |
| Volume [Å³] | 4266.0(2) |
| Z | 8 |
| Density (calculated) | 1.524 |
| Absorption coefficient | 0.395 |
| F(000) | 2048 |
| Crystal size | 0.3 x 0.3 x 0.2 |
| Theta range for data collection | 1.5 → 24 |
| Reflections collected | 14141 |
| Independent reflections | 6383 [Rᵢₙₜ = 0.0713] |
| Data / restraints / parameters | 6383 / 0 / 563 |
| Goodness-of-fit on F² | 1.130 |
| Final R indices [I>2sigma(I)] | R1 = 0.1396, wR2 = 0.3623 |
| R indices (all data) | R1 = 0.1561, wR2 = 0.3773 |

### Example 9: crystalline anhydrous tiotropium hydrogenesulphate (form III)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of sulphuric acid in 10 ml of water. The resulting solution was stored at 4°C in the refrigerator overnight. The next day crystals of a hydrated form of the hydrogenesulphate of tiotropium were formed. These were filtered, washed with cold demineralised water and dried under vacuum.

Approx. 900 mg of the crystalline tiotropium hydrogenesulphate are dissolved in 2 ml of water. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a acetone was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 25 °C. At this temperature the plate remained for a hold time of 24 h. The plates are opened afterwards the solid is obtained by evaporation of the solvent at room temperature in a vacuum chamber (13 kPa). Melting point: 185±5°C (DSC); table 9 summarizes the X-ray powder reflections obtained for this form.

**Table 9:**

| **2 Θ [°]** | **d [Å]** | **I/Iₒ [%]** |
|---|---|---|
| 5.42 | 16.29 | 66 |
| 7.66 | 11.53 | 9 |
| 10.78 | 8.20 | 16 |
| 11.42 | 7.74 | 15 |
| 12.06 | 7.33 | 40 |
| 12.54 | 7.05 | 28 |
| 13.58 | 6.51 | 11 |
| 14.06 | 6.29 | 22 |
| 15.50 | 5.71 | 53 |
| 15.94 | 5.55 | 53 |
| 16.94 | 5.23 | 55 |
| 18.26 | 4.85 | 52 |
| 19.78 | 4.48 | 73 |
| 20.38 | 4.35 | 86 |
| 21.58 | 4.11 | 33 |
| 22.82 | 3.89 | 100 |
| 23.74 | 3.74 | 10 |
| 24.46 | 3.63 | 15 |
| 25.94 | 3.43 | 13 |
| 26.70 | 3.33 | 11 |
| 27.34 | 3.26 | 14 |
| 29.10 | 3.06 | 11 |
| 29.66 | 3.01 | 20 |

### Example 10: crystalline tiotropium dihydrogenephosphate monohydrate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of phosphoric acid in 10 ml of water. The resulting solution was stored at 4°C in the refrigerator overnight. The next day crystals of a hydrated form of the dihydrogenephosphate of tiotropium were formed. These were filtered, washed with cold demineralised water and dried under vacuum. Melting point: 222±3°C (DSC); The crystal structure of this salt was solved by single crystal X-ray diffraction analysis (see table 10a).

**Table 10a:**

| | |
|---|---|
| Empirical formula | C₁₉H₂₂NO₄S₂⁺ • H₂PO₄⁻ • H₂O |
| Fw | 507.5 |
| T [K] | 293(2) |
| λ[Å] | 0.71073 |
| Crystal system | Monoclinic |
| Space group | P 2₁/c |

| Unit cell dimensions | |
|---|---|
| a [Å] | 22.6740(17) A |
| b [Å] | 6.6690(9) Å |
| c [Å] | 15.061(3) Å |
| β[°] | 96.476(8)°. |
| V [Å³] | 2262.9(6) Å³ |
| Z | 4 |
| Dₘ[g/cm³] | 1.490 |
| F(000) | 1064 |
| Crystal size [mm³] | 0.2 x 0.1 x 0.5 |
| θ range[°] | 2 → 22.5. |
| Reflections collected | 4189 |
| Independent reflections | 2509 [Rᵢₙₜ = 0.1336] |
| Data / restraints / parameters | 2509 / 0 / 290 |
| S | 1.039 |
| R [I>2σ(I)] | R1 = 0.0842, wR2 = 0.1859 |
| R indices (all data) | R1 = 0.1632, wR2 = 0.2344 |
| Extinction coefficient | 0.07(2) |

Table 10b summarizes the X-ray powder reflections obtained for this form.

**Table 10b:**

| **2 Θ [°]** | **d [Å]** | **I/Iₒ [%]** |
|---|---|---|
| 4.02 | 21.97 | 44 |
| 7.98 | 11.07 | 15 |
| 12.02 | 7.35 | 78 |
| 13.58 | 6.51 | 56 |
| 14.14 | 6.26 | 81 |
| 15.62 | 5.67 | 59 |
| 16.50 | 5.37 | 30 |
| 17.14 | 5.17 | 22 |
| 18.10 | 4.90 | 97 |
| 18.58 | 4.77 | 64 |
| 19.22 | 4.61 | 67 |
| 20.38 | 4.35 | 40 |
| 21.02 | 4.22 | 100 |
| 22.50 | 3.95 | 58 |
| 23.22 | 3.83 | 31 |
| 24.46 | 3.63 | 76 |
| 25.50 | 3.49 | 20 |
| 26.38 | 3.37 | 19 |
| 27.54 | 3.23 | 38 |
| 28.06 | 3.19 | 20 |
| 28.94 | 3.08 | 16 |
| 29.86 | 2.99 | 18 |

### Example 11: crystalline ditiotropium ethanedisulphonate hydrate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of ethanedisulphonic acid in 10 ml of water. From the resulting solution water was evaporated under vacuum over a water bath which was kept below 35 °C. The obtained solid was further dried under vacuum yielding a hydrated form of the ethanedisulphonate of tiotropium.

The crystal structure of this salt was solved by single crystal X-ray diffraction analysis (see table 11).

**Table 11:**

| | |
|---|---|
| Empirical formula | 2 • (C₁₉H₂₂NO₄S₂⁺) • C₂H₄O₆S₂²⁻ • 10 H₂O |
| Fw | 1152.32 |
| T [K] | 263(2) K |
| λ[Å] | 0.71073 Å |
| Crystal system | Triclinic |
| Space group | P -1 |

| Unit cell dimensions | |
|---|---|
| a [Å] | 9.2700(8) |
| b [Å] | 12.8920(13) |
| c [Å] | 22.579(2) |
| α[°] | 103.876(3) |
| β[°] | 93.620(4) |
| γ[°] | 90.327(5) |
| V [Å³] | 2613.8(4) |
| Z | 2 |
| Dₘ [g/cm³] | 1.464 Mg/m³ |
| F(000) | 1218 |
| Crystal size [mm³] | 0.4 x 0.2 x 0.2 mm3 |
| θ range[°] | 1.5 to 26.5 |
| Reflections collected | 3753 |
| Independent reflections | 3360 [Rᵢₙₜ = 0.0372] |
| Data / restraints / parameters | 3360 / 0 / 292 |
| S | 2.605 |
| R [I>2σ(I)] | R1 = 0.1919, wR2 = 0.4322 |
| R indices (all data) | R1 = 0.2293, wR2 = 0.4495 |
| Extinction coefficient | 0.026(6) |

### Example 12: crystalline tiotropium xinafoate monohydrate (form I)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of 1-hydroxy-2-naphthoic acid in 10 ml of water. From the resulting solution water was evaporated under vacuum over a water bath which was kept below 35 °C. The obtained solid was further dried under vacuum yielding a hydrated form of the xinafoate of tiotropium. Melting point: 170±3°C (DSC): the crystal structure of this salt was solved by single crystal X-ray diffraction analysis analysis (see table 12a).

**Table 12a:**

| | |
|---|---|
| Empirical formula | C₁₉H₂₂NO₄S₂⁺ • C₁₁H₇O₃⁻ • H2O |
| Fw | 597.68 |
| T[K] | 293(2) |
| λ[Å] | 0.71073 |
| Crystal system | Monoclinic |
| Space group | P 2₁/c |

| Unit cell dimensions | |
|---|---|
| a [Å] | 13.5460(16) |
| b [Å] | 16.491(3) |
| c [Å] | 13.263(2) |
| β[°] | 100.51(2) |
| V [Å³] | 2913.0(7) |
| Z | 4 |
| Dₘ[g/cm³] | 1.363 |
| F(000) | 1256 |
| Crystal size [mm³] | 0.3 x 0.3 x 0.2 |
| θ range[°] | 2.5 → 27.5 |
| Reflections collected | 19684 |
| Independent reflections | 6685 [Rᵢₙₜ = 0.0682] |
| Data / restraints / parameters | 6685 / 0 / 406 |
| S | 1.007 |
| R [I>2σ(I)1 | R1 = 0.0586, wR2 = 0.1255 |
| R indices (all data) | R1 = 0.1287, wR2 = 0.1566 |
| Extinction coefficient | 0.0105(12) |

Table 12b summarizes the X-ray powder reflections obtained for this form.

**Table 12b:**

| **2 Θ [°]** | **d [Å]** | **I/Iₒ [%]** |
|---|---|---|
| 6.70 | 13.18 | 25 |
| 8.62 | 10.25 | 27 |
| 10.14 | 8.71 | 9 |
| 10.74 | 8.23 | 20 |
| 11.66 | 7.59 | 7 |
| 13.38 | 6.61 | 16 |
| 14.02 | 6.31 | 34 |
| 14.90 | 5.94 | 63 |
| 16.22 | 5.48 | 8 |
| 17.06 | 5.19 | 18 |
| 18.14 | 4.88 | 17 |
| 19.22 | 4.61 | 14 |
| 20.02 | 4.43 | 8 |
| 20.78 | 4.27 | 14 |
| 21.54 | 4.12 | 100 |
| 22.10 | 4.02 | 9 |
| 22.90 | 3.88 | 28 |
| 23.70 | 3.79 | 7 |
| 24.82 | 3.58 | 15 |
| 26.30 | 3.38 | 13 |
| 27.10 | 3.29 | 11 |
| 27.90 | 3.24 | 10 |
| 28.34 | 3.19 | 9 |
| 28.94 | 3.13 | 5 |
| 29.42 | 3.08 | 3 |

### Example 13: crystalline anhydrous tiotropium xinafoate (form II)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m The collector vial was previously charged with 1.1 equivalents of 1-hydroxy-2-naphthoic acid in 10 ml of water. From the resulting solution water was evaporated under vacuum over a water bath which was kept below 35 °C. The obtained solid was further dried under vacuum yielding a powdery material of the xinafoate of tiotropium.

Approx. 500 mg of the this tiotropium xinafoate are dissolved in 4 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a mixture of diisopropyl ether/methanol = 50:50 was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 25 °C. At this temperature the plate remained for a hold time of 24 h. The plates are opened afterwards the solid is obtained by evaporation of the solvent at room temperature in a vacuum chamber (13 kPa). Melting point by DSC: 180 ± 3 °C; the crystal structure of this salt was solved by single crystal X-ray diffraction analysis (see table 13a).

**Table 13a:**

| | |
|---|---|
| Empirical formula | C₁₉H₂₂NO₇S₂⁺ • C₁₁H₇O₃⁻ |
| Formula weight | 579.66 |
| Temperature | 293(2) |
| Wavelength | 0.71073 |
| Crystal system | Monoclinic |
| Space group | P 2₁/c |

| Unit cell dimensions | |
|---|---|
| a [Å] | 15.9650(4) |
| b [Å] | 13.2330(3) |
| c [Å] | 14.1810(5) |
| β [°] | 111.781(2) |
| Volume [Å³] | 2782.06(14) |
| Z | 4 |
| Density (calculated) | 1.384 |
| F(000) | 1216 |
| Crystal size | 0.45 x 0.3 x 0.2 |
| Theta range for data collection | 3 → 26 |
| Reflections collected | 14803 |
| Independent reflections | 5437 [Rᵢₙₜ = 0.0318] |
| Data / restraints / parameters | 5437/0/411 |
| Goodness-of-fit on F² | 1.043 |
| Final R indices [I>2sigma(I)] | R1 = 0.0631, wR2 = 0.1766 |
| R indices (all data) | R1 = 0.0821, wR2 = 0.1935 |

Table 13b summarizes the X-ray powder reflections obtained for this form.

**Table 13b:**

| **2 Θ [°]** | **d [Å]** | **I/Iₒ [%]** |
|---|---|---|
| 6.06 | 14.57 | 62 |
| 9.86 | 8.96 | 20 |
| 12.58 | 7.03 | 14 |
| 13.50 | 6.55 | 100 |
| 14.78 | 5.99 | 56 |
| 15.90 | 5.57 | 30 |
| 17.22 | 5.14 | 25 |
| 18.06 | 4.91 | 69 |
| 19.30 | 4.59 | 36 |
| 20.54 | 4.32 | 12 |
| 21.46 | 4.14 | 32 |
| 22.66 | 3.92 | 64 |
| 23.30 | 3.81 | 16 |
| 23.98 | 3.79 | 5 |
| 25.50 | 3.49 | 37 |
| 26.46 | 3.36 | 13 |
| 27.18 | 3.28 | 27 |
| 28.26 | 3.15 | 11 |
| 28.90 | 3.12 | 7 |
| 29.70 | 3.00 | 10 |

### Example 14: crystalline tiotropium xinafoate monohydrate (form III)

6.00 g of tiotropium bromide monohydrate was dissolved in 350 ml of demineralised water at 25°C. 150 ml of bicarbonate loaded IER slurry was added to this solution and stirred for 5 minutes. A silver nitrate test as described above indicated the completion of the exchange. After stirring for an additional 5 minutes the slurry was filtered with PTFE filters with a pore size of 10 µm and washed with 150 ml water. 1.1 equivalents of 1-hydroxy-2-naphthoic acid in 70 ml isopropanol were added to the filtrate. From the resulting solution water and isopropanol was evaporated under gentle conditions (40 - 50 °C). To the obtained oil first 50 ml isopropanol and than 40 ml ethanol was added and heated up to 70 °C. A clear slightly brownish solution was obtained which was filtered with charcoal. The obtained clear solution was stirred at room temperature. After 72 hours stirring at room temperature a suspension was obtained. the crystals were filtered off, washed with a small amount of cold isopropanol and than dried over night at room temperature. Melting point by DSC: 110 ± 3 °C (under dehydration); the crystal structure of this salt was solved by single crystal X-ray diffraction analysis. (see table 14a).

**Table 14a:**

| | |
|---|---|
| Empirical formula | C₁₉H₂₂NO₄S₂⁺ • C₁₁H₇O₃⁻ • H₂O |
| Formula weight | 597.68 |
| Temperature | 293(2) |
| Wavelength | 0.71073 |
| Crystal system | Monoclinic |
| Space group | P 2₁/c |

| Unit cell dimensions | |
|---|---|
| a [Å] | 13.2470(6) |
| b [Å] | 11.3590(6) |
| c [Å] | 20.9500(7) |
| β [°] | 118.229(4) |
| Volume [Å³] | 2777.5(2) |
| Z | 4 |
| Density (calculated) | 1.429 |
| F(000) | 1256 |
| Crystal size | 0.45 x 0.3 x 0.25 |
| Theta range for data collection | 2 → 25. |
| Reflections collected | 3999 |
| Independent reflections | 3092 [Rᵢₙₜ = 0.0568] |
| Data / restraints / parameters | 3092 / 0 / 375 |
| Goodness-of-fit on F2 | 1.889 |
| Final R indices [I>2sigma(I)] | R1 = 0.1001, wR2 = 0.2694 |
| R indices (all data) | R1 = 0.1208, wR2 = 0.2837 |

Table 14b summarizes the X-ray powder reflections obtained for this form.

**Table 14b:**

| **2 Θ [°]** | **d [Å]** | **I/Iₒ [%]** |
|---|---|---|
| 8.93 | 9.90 | 73 |
| 9.13 | 9.68 | 10 |
| 9.55 | 9.26 | 5 |
| 10.32 | 8.57 | 8 |
| 10.84 | 8.16 | 45 |
| 11.84 | 7.47 | 11 |
| 12.32 | 7.18 | 26 |
| 13.23 | 6.69 | 16 |
| 13.56 | 6.53 | 4 |
| 14.79 | 5.99 | 49 |
| 15.16 | 5.84 | 13 |
| 15.59 | 5.68 | 19 |
| 16.31 | 5.43 | 37 |
| 17.04 | 5.20 | 86 |
| 17.94 | 4.94 | 3 |
| 18.28 | 4.85 | 14 |
| 18.64 | 4.76 | 27 |
| 19.15 | 4.63 | 16 |
| 19.55 | 4.54 | 85 |
| 20.09 | 4.42 | 100 |
| 20.70 | 4.29 | 45 |
| 21.49 | 4.13 | 13 |
| 21.81 | 4.07 | 37 |
| 22.84 | 3.89 | 17 |
| 23.08 | 3.85 | 12 |
| 23.84 | 3.73 | 10 |
| 24.42 | 3.65 | 3 |
| 24.78 | 3.59 | 51 |
| 25.07 | 3.55 | 12 |
| 25.32 | 3.52 | 13 |
| 26.62 | 3.35 | 23 |
| 27.28 | 3.27 | 30 |
| 27.72 | 3.22 | 5 |
| 28.02 | 3.18 | 10 |
| 28.75 | 3.10 | 20 |
| 29.61 | 3.02 | 10 |
| 29.93 | 2.98 | 4 |

### Example 15: crystalline tiotropium fumarate ethanolate (form I)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of fumaric acid in 10 ml of water. From the resulting solution water was evaporated under vacuum over a water bath which was kept below 35 °C. The obtained solid was further dried under vacuum yielding an amorphous oil of the fumarate of tiotropium. This material was recrystallised afterwards from ethanol as followed. About 100 mg of the salt was suspended in absolute ethanol. The mixture was heated to 50°C and kept at that temperature for approximately 1h until complete dissolution was observed. The solution was not filtered and cooled slowly to room temperature. After several days, crystals of a solvated form containing ethanol of the fumarate of tiotropium were formed, which were isolated by filtration.

The crystal structure of this salt was solved by single crystal X-ray diffraction analysis (see table 15).

**Table 15:**

| | |
|---|---|
| Empirical formula | C₁₉H₂₂NO₄S₂⁺ • C₄H₃O₄⁻ • C₂H₆O |
| Fw | 553.63 |
| T [K] | 293(2) |
| λ[Å] | 0.71073 |
| Crystal system | Orthorhombic |
| Space group | P bca |

| Unit cell dimensions | |
|---|---|
| a [Å] | 15.3830(7) |
| b [Å] | 16.8490(7) |
| c [Å] | 20.0900(12) |
| V [Å³] | 5207.1(4) |
| Z | 8 |
| Dₘ [g/cm³] | 1.412 |
| F(000) | 2336 |
| Crystal size [mm³] | 0.2 x 0.2 x 0.08 |
| θ range[°] | 3 → 22.5. |
| Reflections collected | 6183 |
| Independent reflections | 3285 [Rᵢₙₜ = 0.0458] |
| Data / restraints / parameters | 3285/0/336 |
| S | 1.040 |
| R [I>2σ(I)] | R1 = 0.0923, wR2 = 0.2229 |
| R indices (all data) | R1 = 0.1243, wR2 = 0.2489 |
| Extinction coefficient | 0.0020(9) |

### Example 16: crystalline anhydrous tiotropium fumarate (form II)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of fumaric acid in 10 ml of water. From the resulting solution water was evaporated under vacuum over a water bath which was kept below 35 °C. The obtained solid was further dried under vacuum yielding an amorphous oil of the fumarate of tiotropium. This material was recrystallised afterwards from ethanol as followed. About 100 mg of the salt was suspended in absolute ethanol. The mixture was heated to 50°C and kept at that temperature for approximately 1h until complete dissolution was observed. The solution was not filtered and cooled slowly to room temperature. After several weeks, large crystals of an anhydrous fumarate of tiotropium were formed, which were isolated by filtration. Melting point: 180±3°C (DSC); The crystal structure of this salt was solved by single crystal X-ray diffraction analysis (see table 16a).

**Table 16a:**

| | |
|---|---|
| Empirical formula | C₁₉H₂₂NO₄S₂⁺ • C₄H₃O₄⁻ |
| Fw | 507.56 |
| T [K] | 293(2) K |
| λ[Å] | 0.71073 Å |
| Crystal system | Triclinic |
| Space group | P-1 |

| Unit cell dimensions | |
|---|---|
| a [Å] | 7.4980(3) |
| b [Å] | 9.4900(4) |
| c [Å] | 17.0110(7) |
| α[°] | 102.125(2) |
| β[°] | 96.182(2) |
| γ[°] | 99.289(2) |
| V [Å³] | 1155.27(8) |
| Z | 2 |
| Dₘ [g/cm³] | 1.459 |
| F(000) | 532 |
| Crystal size [mm³] | 0.45 x 0.4 x 0.2 |
| θ range[°] | 2.3 → 27.7 |
| Reflections collected | 6544 |
| Independent reflections | 4796 [Rᵢₙₜ= 0.0354] |
| Data / restraints / parameters | 4796/0/410 |
| S | 1.023 |
| R [I>2σ(I)] | R1 = 0.0493, wR2 = 0.1201 |
| R indices (all data) | R1 = 0.0646, wR2 = 0.1322 |

Table 16b summarizes the X-ray powder reflections obtained for this form.

**Table 16b:**

| **2 Θ [°]** | **d [Å]** | **I/Iₒ [%]** |
|---|---|---|
| 6.30 | 14.01 | 4 |
| 10.06 | 8.78 | 47 |
| 10.86 | 8.14 | 11 |
| 12.50 | 7.07 | 64 |
| 14.14 | 6.26 | 88 |
| 15.14 | 5.84 | 45 |
| 16.26 | 5.44 | 42 |
| 18.02 | 4.92 | 77 |
| 18.86 | 4.70 | 100 |
| 19.58 | 4.53 | 58 |
| 21.06 | 4.21 | 63 |
| 21.78 | 4.08 | 77 |
| 22.42 | 3.96 | 61 |
| 24.38 | 3.65 | 48 |
| 25.02 | 3.55 | 53 |
| 25.70 | 3.46 | 42 |
| 26.74 | 3.33 | 13 |
| 28.34 | 3.15 | 36 |
| 28.86 | 3.09 | 23 |
| 29.78 | 3.00 | 16 |

### Example 17: crystalline anhydrous tiotropium fumarate (form III)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of fumaric acid in 10 ml of water. From the resulting solution water was evaporated under vacuum over a water bath which was kept below 35 °C. The obtained solid was further dried under vacuum yielding an amorphous oil of the fumarate of tiotropium.

Approx. 600 mg of the amorphous tiotropium fumarate are dissolved in 4 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of 1-methyl-2-pyrollidinone (= NMP, extra dry) was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 25°C. At this temperature the plate remained for a hold time of 24 h. The plates are opened afterwards the solid is obtained by evaporation of the solvent at room temperature in a vacuum chamber (13 kPa). Table 17 summarizes the X-ray powder reflections obtained for this form.

**Table 17:**

| **2 Θ [°]** | **d [Å]** | **I/Iₒ [%]** |
|---|---|---|
| 9.02 | 9.79 | 21 |
| 10.02 | 8.82 | 35 |
| 10.82 | 8.17 | 9 |
| 12.42 | 7.12 | 51 |
| 13.46 | 6.57 | 11 |
| 14.06 | 6.29 | 65 |
| 15.14 | 5.84 | 46 |
| 16.22 | 5.46 | 48 |
| 16.82 | 5.26 | 37 |
| 17.94 | 4.94 | 69 |
| 18.82 | 4.71 | 68 |
| 19.54 | 4.54 | 100 |
| 21.02 | 4.22 | 49 |
| 21.66 | 4.10 | 68 |
| 22.38 | 3.97 | 46 |
| 23.74 | 3.74 | 32 |
| 24.26 | 3.66 | 46 |
| 24.82 | 3.58 | 50 |
| 25.66 | 3.47 | 44 |
| 26.22 | 3.39 | 29 |
| 28.26 | 3.15 | 25 |
| 28.94 | 3.08 | 24 |
| 29.70 | 3.00 | 17 |
| 30.34 | 2.94 | 22 |

### Example 18: crystalline anhydrous tiotropium fumarate (form IV)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of fumaric acid in 10 ml of water. From the resulting solution water was evaporated under vacuum over a water bath which was kept below 35 °C. The obtained solid was further dried under vacuum yielding an amorphous oil of the fumarate of tiotropium.

Approx. 600 mg of the amorphous tiotropium fumarate are dissolved in 4 ml of a mixture of acetone/water = 80:20. In two steps 75 *µ*l (40 + 35 *µ*l) of this stock solution is transferred into one of the small vials of a 96 well plate: The plate containing the stock solution was placed in a vacuum chamber (1 kPa) at room temperature for 24 h for each dosing step. After the stock solvent was evaporated 30 *µ*l of a mixture of acetonitrie/water = 50:50 was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 5 °C. At this temperature the plate remained for a hold time of 24 h. The plates are opened afterwards the solid is obtained by evaporation of the solvent at room temperature in a vacuum chamber (13 kPa). Melting point: 225±3°C (DSC); table 18a summarizes the X-ray powder reflections obtained for this form.

**Table 18a:**

| **2 Θ [°]** | **d [Å]** | **I/Iₒ [%]** |
|---|---|---|
| 8.18 | 10.80 | 10 |
| 10.22 | 8.65 | 18 |
| 11.10 | 7.96 | 84 |
| 12.70 | 6.96 | 12 |
| 13.38 | 6.61 | 53 |
| 15.22 | 5.81 | 34 |
| 16.54 | 5.35 | 64 |
| 17.58 | 5.04 | 35 |
| 18.46 | 4.80 | 100 |
| 19.10 | 4.64 | 76 |
| 20.54 | 4.32 | 35 |
| 21.42 | 4.14 | 50 |
| 21.94 | 4.05 | 58 |
| 22.42 | 3.96 | 77 |
| 23.86 | 3.73 | 6 |
| 24.58 | 3.63 | 4 |
| 25.42 | 3.50 | 15 |
| 26.14 | 3.40 | 18 |
| 26.82 | 3.32 | 20 |
| 27.30 | 3.26 | 15 |
| 27.70 | 3.22 | 46 |
| 28.14 | 3.17 | 20 |
| 28.86 | 3.09 | 15 |
| 29.62 | 3.01 | 20 |

A single crystal x-ray structure analysis of form IV of tiotropium fumarate showed that this form is in fact a co-crystal of a 2:1 salt (tiotropium: counter ion) of tiotropium fumarate which contains an additional mol of fumaric acid.

The crystal structure of this salt was solved by single crystal X-ray diffraction analysis (see table 18b).

**Table 18b:**

| | |
|---|---|
| Empirical formula | 2 C₁₉H₂₂NO₄S₂⁺ • C₄H₂O₄²⁻• C₂H₄O₄ |
| Fw | 1015.12 |
| T [K] | 293(2) |
| λ[Å] | 0.71073 |
| Crystal system | Monoclinic |
| Space group | P 2₁/c |

| Unit cell dimensions | |
|---|---|
| a [Å] | 9.6910(2) |
| b [Å] | 14.5710(4) |
| c [Å] | 18.1580(4) |
| β [°] | 116.781(2) |
| V [Å³] | 2289.01 (9) |
| Z | 2 |
| Dₘ [g/cm³] | 1.473 |
| F(000) | 1064 |
| Crystal size [mm³] | 0.4 x 0.4 x 0.2 |
| θ range [°] | 3 → 26 |
| Reflections collected | 11429 |
| Independent reflections | 4424 [Rᵢₙₜ = 0.0352] |
| Data / restraints / parameters | 4424/0/424 |
| S | 1.030 |
| R [I>2σ(I)] | R1 = 0.0459, wR2 = 0.1070 |
| R indices (all data) | R1 = 0.0630, wR2 = 0.1178 |

### Example 19: crystalline anhydrous tiotropium tartrate (form I)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional. 5 minutes the slurry was filtered with PTFE filters with a pore size of 10 *µ*m. The collector vial was previously charged with 1.1 equivalents of D-tartaric acid in 10 ml of water. From the resulting solution water was evaporated under vacuum over a water bath which was kept below 35 °C. The obtained solid was further dried under vacuum yielding an amorphous oil of the tartrate of tiotropium.

Approx. 500 mg of the amorphous tiotropium tartrate are dissolved in 4 ml of a water. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a mixture of acetonitrile/water= 50:50 was added to this vial. The whole 96 well plate is sealed afterwards and heated up with a heating rate of 5 °C/min to 50°C at which the plate stays for an additional 30 minutes. Afterwards the plate is cooled with a cooling rate of 5°C/h to a final temperature of 5 °C. At this temperature the plate remained for a hold time of 24 h. The plates are opened afterwards the solid is obtained by evaporation of the solvent at room temperature in a vacuum chamber (13 kPa). Melting point: 180±3°C (DSC); table 19 summarizes the X-ray powder reflections obtained for this form.

**Table 19:**

| **2 Θ [°]** | **d [Å]** | **I/Iₒ [%]** |
|---|---|---|
| 4.38 | 20.16 | 100 |
| 8.74 | 10.11 | 24 |
| 10.78 | 8.20 | 27 |
| 12.18 | 7.25 | 15 |
| 12.82 | 6.90 | 38 |
| 13.90 | 6.36 | 6 |
| 14.46 | 6.12 | 16 |
| 16.22 | 5.46 | 58 |
| 17.42 | 5.08 | 66 |
| 17.98 | 4.93 | 38 |
| 18.86 | 4.70 | 14 |
| 20.10 | 4.41 | 99 |
| 21.58 | 4.11 | 20 |
| 22.14 | 4.02 | 11 |
| 23.66 | 3.76 | 7 |
| 24.38 | 3.65 | 11 |
| 25.90 | 3.44 | 4 |
| 26.46 | 3.36 | 9 |

### Example 20: crystalline anhydrous tiotropium tartrate (form II)

1.00 g of tiotropium bromide monohydrate (according, to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of D-tartaric acid in 10 ml of water. From the resulting solution water was evaporated under vacuum over a water bath which was kept below 35 °C. The obtained solid was further dried under vacuum yielding an amorphous oil of the tartrate of tiotropium.

Approx. 500 mg of the amorphous tiotropium tartrate are dissolved in 4 ml of a water. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of ethanol was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 25 °C. At this temperature the plate remained for a hold time of 24 h. The plates are opened afterwards the solid is obtained by evaporation of the solvent at room temperature in a vacuum chamber (13 kPa). Melting point: 190±3°C (DSC); table 20 summarizes the X-ray powder reflections obtained for this form.

**Table 20:**

| **2 Θ [°]** | **d [Å]** | **I/Iₒ [%]** |
|---|---|---|
| 5.62 | 15.71 | 61 |
| 9.42 | 9.38 | 93 |
| 11.22 | 7.88 | 28 |
| 12.82 | 6.90 | 23 |
| 14.82 | 5.97 | 47 |
| 15.78 | 5.61 | 17 |
| 16.02 | 5.53 | 21 |
| 16.82 | 5.26 | 76 |
| 18.14 | 4.88 | 100 |
| 19.02 | 4.66 | 21 |
| 20.26 | 4.38 | 45 |
| 21.58 | 4.11 | 70 |
| 22.26 | 3.99 | 45 |
| 22.82 | 3.89 | 36 |
| 24.18 | 3.68 | 39 |
| 24.78 | 3.59 | 14 |
| 25.54 | 3.48 | 18 |
| 26.78 | 3.33 | 18 |
| 27.22 | 3.28 | 8 |
| 28.58 | 3.12 | 14 |
| 29.30 | 3.04 | 22 |
| 29.94 | 2.98 | 14 |

### Example 21: crystalline anhydrous tiotropium tartrate (form III)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of D-tartaric acid in 10 ml of water. From the resulting solution water was evaporated under vacuum over a water bath which was kept below 35 °C. The obtained solid was further dried under vacuum yielding an amorphous oil of the tartrate of tiotropium.

Approx. 500 mg of the amorphous tiotropium tartrate are dissolved in 4 ml of a water. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a mixture of 1-methyl-2-pyrrolidinon (= NMP) was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 5 °C. At this temperature the plate remained for a hold time of 24 h. The plates are opened afterwards the solid is obtained by evaporation of the solvent at room temperature in a vacuum chamber (13 kPa).

Table 21 summarizes the X-ray powder reflections obtained for this form.

**Table 21:**

| **2 Θ [°]** | **d [Å]** | **I/Iₒ [%]** |
|---|---|---|
| 8.54 | 10.34 | 35 |
| 12.42 | 7.12 | 24 |
| 13.14 | 6.73 | 35 |
| 13.90 | 6.36 | 49 |
| 14.46 | 6.12 | 47 |
| 15.82 | 5.60 | 37 |
| 16.22 | 5.46 | 25 |
| 17.06 | 5.19 | 62 |
| 17.58 | 5.04 | 100 |
| 18.30 | 4.84 | 30 |
| 19.06 | 4.65 | 31 |
| 20.22 | 4.39 | 16 |
| 21.90 | 4.05 | 54 |
| 22.66 | 3.92 | 11 |
| 23.70 | 3.76 | 14 |
| 24.86 | 3.58 | 28 |
| 25.66 | 3.49 | 14 |
| 27.42 | 3.25 | 26 |
| 27.98 | 3.19 | 14 |
| 28.82 | 3.09 | 7 |
| 29.46 | 3.03 | 11 |

### Example 22: crystalline anhydrous tiotropium succinate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of succinic acid in 10 ml of water. From the resulting solution water was evaporated under vacuum over a water bath which was kept below 35 °C. The obtained solid was further dried under vacuum yielding an amorphous oil of the succinate of tiotropium.

Approx. 600 mg of the amorphous tiotropium succinate are dissolved in 4 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of 1-butanol was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to a final temperature of 5 °C. At this temperature the plate remained for a hold time of 24 h. The plates are opened afterwards the solid is obtained by evaporation of the solvent at room temperature in a vacuum chamber (13 kPa). Melting point: 193±3°C (DSC); table 22 summarizes the X-ray powder reflections obtained for this form.

**Table 22:**

| **2 Θ [°]** | **d [Å]** | **I/Iₒ [%]** |
|---|---|---|
| 8.22 | 10.74 | 14 |
| 10.42 | 8.48 | 13 |
| 11.18 | 7.90 | 53 |
| 13.34 | 6.63 | 19 |
| 14.98 | 5.91 | 24 |
| 16.50 | 5.37 | 23 |
| 17.38 | 5.10 | 34 |
| 18.74 | 4.73 | 100 |
| 20.50 | 4.33 | 17 |
| 21.74 | 4.08 | 92 |
| 22.58 | 3.93 | 63 |
| 24.02 | 3.71 | 6 |
| 25.18 | 3.53 | 7 |
| 25.70 | 3.47 | 8 |
| 26.74 | 3.33 | 13 |
| 28.14 | 3.17 | 85 |
| 29.90 | 2.98 | 25 |

### Example 23: crystalline anhydrous tiotropium malonate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of malonic acid in 10 ml of water. The salt solution was concentrated to about 15 - 20 ml, frozen in a 100ml jar at - 20°C and placed in the freeze dryer at a final pressure of < 0.05mbar (over night). A white fluffy cake that could be loosened-up easily was the result.

Approx. 500 mg of the amorphous tiotropium malonate are dissolved in 5 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a ethylene glycol was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to 30 °C and than with cooling rate of 1 °C/min to a final temperature of 5 °C. At this temperature the plate remained for a hold time of 24 h. The plates are opened afterwards the solid is obtained by evaporation of the solvent at room temperature in a vacuum chamber (13 kPa). Table 23 summarizes the X-ray powder reflections obtained for this form.

**Table 23:**

| **2 Θ [°]** | **d [Å]** | **I/Iₒ [%]** |
|---|---|---|
| 7.30 | 12.10 | 15 |
| 7.82 | 11.29 | 5 |
| 8.58 | 10.29 | 24 |
| 11.34 | 7.79 | 9 |
| 12.22 | 7.23 | 11 |
| 13.26 | 6.67 | 97 |
| 13.82 | 6.40 | 13 |
| 15.02 | 5.89 | 10 |
| 17.22 | 5.14 | 74 |
| 18.02 | 4.92 | 79 |
| 19.42 | 4.57 | 98 |
| 20.22 | 4.39 | 97 |
| 21.50 | 4.13 | 20 |
| 23.26 | 3.82 | 13 |
| 23.78 | 3.74 | 7 |
| 24.74 | 3.59 | 8 |
| 25.42 | 3.50 | 29 |
| 25.94 | 3.43 | 8 |
| 26.82 | 3.32 | 54 |
| 27.34 | 3.26 | 7 |
| 27.94 | 3.19 | 14 |
| 28.38 | 3.14 | 11 |
| 29.42 | 3.03 | 11 |

### Example 24: crystalline tiotropium malate N,N-dimethylacetamide (DMA) solvate (form I)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of L-malic acid in 10 ml of water. The salt solution was concentrated to about 15 - 20 ml, frozen in a 100ml jar at-20°C and placed in the freeze dryer at a final pressure of < 0.05mbar (over night). A white fluffy cake that could be loosened-up easily was the result.

Approx. 500 mg of the amorphous tiotropium malate are dissolved in 5 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a N,N-dimethyl-acetamide was added to this vial. The whole 96 well plate is sealed afterwards and heated up with a heating rate of 5 °C/min to 50 °C at which the plate stays for an additional 30 minutes. Afterwards the plate is cooled with a cooling rate of 5°C/h to 30 °C and than with cooling rate of 1 °C/min to a final temperature of 5 °C. At this temperature the plate remained for a hold time of 24 h. The plates are opened afterwards the solid is obtained by evaporation of the solvent at room temperature in a vacuum chamber (13 kPa). Melting point: 170±5°C (DSC; under decomposition): the crystal structure of this salt was solved by single crystal X-ray diffraction analysis (see table 24).

**Table 24:**

| | |
|---|---|
| Empirical formula | C₁₉H₂₂NO₄S₂⁺ • C₄H₅O₅⁻ • C₄H₉NO |
| Fw | 612.70 |
| T [K] | 293(2) |
| λ[Å] | 0.71073 |
| Crystal system | Monoclinic |
| Space group | P2₁ |

| Unit cell dimensions | |
|---|---|
| a [Å] | 7.4670(5) |
| b [Å] | 14.4950(9) |
| c [Å] | 14.0490(14) |
| ß | 100.095(2) |
| V [Å³] | 1497.0(2) |
| Z | 2 |
| Dₘ [g/cm³] | 1.359 |
| F(000) | 648 |
| Crystal size [mm³] | 0.3 x 0.25 x 0.2 |
| θ range[°] | 2.77 → 27.48 |
| Reflections collected | 4633 |
| Independent reflections | 3810 [Rᵢₙₜ = 0.0659] |
| Data / restraints / parameters | 3810 / 1 / 382 |
| S | 1.078 |
| R [I<2σ(I)] | R1 = 0.0678, wR2 = 0.1441 |
| R indices (all data) | R1 = 0.1085, wR2 = 0.1657 |
| Absolute structure parameter (Flack parameter) | 0.14(17) |

### Example 25: crystalline tiotropium malate 1-methyl-2-pyrollidinone (NMP) solvate (form II)

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m.

The collector vial was previously charged with 1.1 equivalents of L-malic acid in 10 ml of water. The salt solution was concentrated to about 15 - 20 ml, frozen in a 100ml jar at - 20°C and placed in the freeze dryer at a final pressure of < 0.05mbar (over night). A white fluffy cake that could be loosened-up easily was the result.

Approx. 500 mg of the amorphous tiotropium malate are dissolved in 5 ml of a mixture of acetone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a NMP/methanol = 60:40 was added to this vial. The whole 96 well plate is sealed afterwards and heated up with a heating rate of 5 °C/min to 50°C at which the plate stays for an additional 30 minutes. Afterwards the plate is cooled with a cooling rate of 5°C/h to 30 °C and than with cooling rate of 1 °C/min to a final temperature of 5 °C. At this temperature the plate remained for a hold time of 24 h. The plates are opened afterwards the solid is obtained by evaporation of the solvent at room temperature in a vacuum chamber (13 kPa). Melting point: 170±5°C (DSC; under decomposition); table 25 summarizes the X-ray powder reflections obtained for this form.

**Table 25:**

| **2 Θ [°]** | **d [Å]** | **I/Iₒ [%]** |
|---|---|---|
| 6.46 | 13.67 | 26 |
| 8.86 | 9.97 | 53 |
| 12.18 | 7.26 | 25 |
| 13.66 | 6.47 | 100 |
| 14.14 | 6.26 | 38 |
| 14.94 | 5.92 | 84 |
| 15.70 | 5.69 | 22 |
| 16.14 | 5.49 | 36 |
| 16.86 | 5.25 | 54 |
| 17.30 | 5.12 | 68 |
| 17.62 | 5.09 | 53 |
| 19.30 | 4.59 | 40 |
| 20.06 | 4.49 | 35 |
| 21.46 | 4.14 | 23 |
| 22.42 | 3.96 | 43 |
| 22.90 | 3.88 | 27 |
| 23.74 | 3.74 | 25 |
| 24.50 | 3.63 | 42 |
| 25.34 | 3.51 | 64 |
| 25.78 | 3.45 | 41 |
| 26.74 | 3.33 | 15 |
| 27.62 | 3.23 | 16 |
| 28.82 | 3.09 | 15 |
| 29.42 | 3.03 | 8 |

### Example 26: crystalline tiotropium oxalate dihydrate

1.00 g of tiotropium bromide monohydrate (according to WO 02/30928) was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After shirring for an additional 5 minutes the slurry was filtered with PTFE filters with a pore size of 10 *µ*m. The collector vial was previously charged with 1.1 equivalents of oxalic acid in 10 ml of water. The salt solution was concentrated to about 15-20 ml, frozen in a 100ml jar at-20°C and placed in the freeze dryer at a final pressure of < 0.05mbar (over night). A white fluffy cake that could be loosened-up easily was the result.

Approx. 800 mg of the amorphous tiotropium oxalate are dissolved in 5 ml of a mixture of acctone/water = 80:20. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a mixture of 1,2-dimethoxyethane/ methanol 60:40 was added to this vial. The whole 96 well plate is sealed afterwards and heated up with a heating rate of 5 °C/min to 50 °C at which the plate stays for an additional 30 minutes. Afterwards the plate is cooled with a cooling rate of 10°C/h to a final temperature of 5 °C. At this temperature the plate remained for a hold time of 24 h. The plates arc opened afterwards the solid is obtained by evaporation of the solvent at room temperature in a vacuum chamber (13 kPa).

The crystal structure of this salt was solved by single crystal X-ray diffraction analysis (see table 26).

**Table 26:**

| | |
|---|---|
| Empirical formula | C₁₉H₂₂NO₄S₂⁺ • C₂O₄H⁻ • 2 H₂O |
| Fw | 517.56 |
| T [K] | 293(2) |
| λ[Å] | 0.71073 |
| Crystal system | Monoclinic |
| Space group | P 2₁/c |

| Unit cell dimensions | |
|---|---|
| a [Å] | 11.4540(4) |
| b [Å] | 10.0620(4) |
| c [Å] | 20.2480(9) |
| β [°] | 95.969(2) |
| V [Å3] | 2320.93(16) |
| Z | 4 |
| Dₘ (g/cm³]. | 1.481 |
| F(000) | 1088 |
| Crystal size [mm3] | 0.3 x 0.3 x 0.1 |
| θ range[°] | 2 → 28.5°. |
| Reflections collected | 5184 |
| Independent reflections | 5184 [Rᵢₙₜ = 0.0000*] |
| Data / restraints / parameters | 5184/0/331 |
| S | 1.153 |
| R [I>2σ(I)] | R1 = 0.1009, wR2 = 0.2111 |
| R indices (all data) | R1 = 0.1325, wR2 = 0.2315 |

### Example 27: crystalline anhydrous tiotropium p-toluenesulphonate (form II)

1.00 g of tiotropium bromide monohydrate was dissolved in 50 ml of demineralised water at 25°C. 10 ml of bicarbonate loaded IER slurry was added to this solution and stirred for 5 minutes before a second equivalent of 5 ml bicarbonate loaded IER slurry was added. The mixture was stirred for additional 5 minutes. A silver nitrate test as described above indicated the completion of the exchange. After stirring for an additional 5 minutes the slurry was filtered with PTFE filters with a pore size of 10 *µ*m. The collector vial was previously charged with 1.1 equivalents of p-toluenesulfonic acid in 10 ml of water. From the resulting solution water was evaporated under vacuum over a water bath which was kept below 35 °C. The obtained solid was further dried under vacuum yielding an amorphous oil of the tosylate of tiotropium. The amorphous oil was dissolved in acetonitril/water (70:30) and freeze dried over night. An amorphous solid material was obtained.

Approx. 480 mg of the amorphous tiotropium tosylate are dissolved in 10 ml of a mixture of acetonitrile/water = 70:30. In two steps 75 *µ*l (40 + 35 *µ*l) of this stock solution is transferred into one of the small vials of a 96 well plate. The plate containing the stock solution was placed in a vacuum chamber (1 kPa) at room temperature for 24 h for each dosing step. After the stock solvent was evaporated 30 *µ*l of a chloroform was added to this vial. The whole 96 well plate is sealed afterwards and heated up with a heating rate of 5 °C/min to 50 °C at which the plate stays for an additional 30 minutes. Afterwards the plate is cooled with a cooling rate of 30°C/h to 3 °C. At this temperature the plate remained for a hold time of 1 h. The plates are opened afterwards the solid is obtained by evaporation of the solvent at room temperature in a vacuum chamber (13 kPa). Melting point: 140 ± 5 °C (DSC): table 27 summarizes the X-ray powder reflections obtained for this form.

**Table 27:**

| **2 Θ [°]** | **d [Å]** | **I/Iₒ [%]** |
|---|---|---|
| 4.74 | 18.64 | 14 |
| 9.62 | 9.19 | 15 |
| 11.02 | 8.03 | 19 |
| 11.74 | 7.54 | 37 |
| 13.50 | 6.56 | 36 |
| 14.26 | 6.21 | 8 |
| 15.22 | 5.82 | 11 |
| 16.10 | 5.50 | 12 |
| 16.58 | 5.35 | 6 |
| 17.46 | 5.08 | 100 |
| 18.10 | 4.90 | 20 |
| 18.78 | 4.72 | 26 |
| 19.82 | 4.48 | 47 |
| 20.50 | 4.33 | 7 |
| 21.42 | 4.15 | 26 |
| 22.10 | 4.02 | 11 |
| 22.54 | 3.94 | 18 |
| 23.18 | 3.84 | 71 |
| 24.02 | 3.70 | 12 |
| 25.94 | 3.43 | 6 |
| 26.54 | 3.36 | 9 |
| 27.30 | 3.27 | 26 |
| 28.14 | 3.17 | 10 |
| 28.98 | 3.08 | 8 |
| 29.18 | 3.06 | 6 |
| 29.90 | 2.99 | 11 |

### Example 28: crystalline^{:} tiotropium methanesulphonate monohydrate (form II)

Approximately 60 mg of the crystalline tiotropium methanesulphonate (anhydrous form I disclosed in WO 05/042528) are suspended in 2 ml of diisobutyl ketone. The slurry was stirred for 30 minutes at 60 °C. Afterwards the solvent was gently evaporated at room temperature in a vacuum chamber (13 kPa). The crystalline material obtained was tiotropium methanesulphonate, form II which is hydrated form (monohydrate) of tiotropium methanesulphonate. Melting point: 235 ± 3 °C (DSC); table 28 summarizes the X-ray powder reflections obtained for this form.

**Table 28:**

| **2 Θ [°]** | **d [Å]** | **I/Iₒ [%]** |
|---|---|---|
| 10.22 | 8.65 | 3 |
| 10.94 | 8.09 | 3 |
| 12.34 | 7:17 | 21 |
| 13.18 | 6.72 | 15 |
| 14.02 | 6.32 | 31 |
| 15.00 | 5.91 | 16 |
| 15.70 | 5.64 | 11 |
| 15.90 | 5.57 | 6 |
| 16.34 | 5.42 | 13 |
| 17.10 | 5.18 | 25 |
| 17.90 | 4.95 | 26 |
| 18.44 | 4.81 | 22 |
| 19.86 | 4.47 | 100 |
| 21.74 | 4.09 | 47 |
| 22.50 | 3.95 | 12 |
| 22.82 | 3.90 | 12 |
| 23.50 | 3.79 | 39 |
| 25.14 | 3.54 | 35 |
| 26.66 | 3.34 | 27 |
| 27.90 | 3.20 | 21 |
| 28.22 | 3.16 | 30 |
| 29.46 | 3.03 | 17 |
| 30.06 | 2.97 | 9 |
| 31.66 | 2.83 | 23 |
| 32.18 | 2.78 | 24 |

## Claims

1. Process for preparing new tiotropium salts of formula **1** 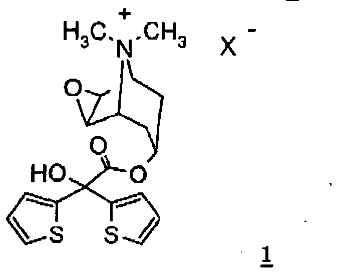 wherein
X⁻ denotes an anion which is different from HCO₃⁻ (=bicarbonate)
**characterised in that** tiotropium bicarbonate of formula **2** 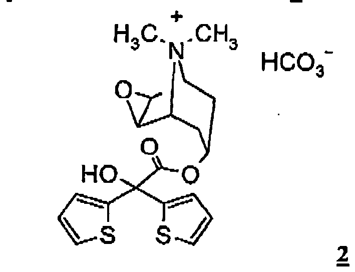 is reacted in a suitable solvent with an acid HX wherein X may have the meanings given above.

2. Process according to claim 1, wherein
X⁻ denotes halide, HSO₄⁻, H₂PO₄⁻ or an anion selected from optionally substituted alkylsulphonate, optionally substituted alkenylsulphonate, optionally substituted alkinylsulphonate, optionally substituted cycloalkylsulphonate, optionally substituted alkylsulphate, optionally substituted alkenylsulphate, optionally substituted alkinylsulphate, optionally substituted cycloalkylsulphate, optionally substituted arylsulphonate, optionally substituted arylsulphate, optionally substituted heterocyclylsulphonate and optionally substituted heterocyclylsulphate;
or
X⁻ denotes R-COO⁻, wherein R is hydrogen, COOH, COO-C₁-C₆-alkyl or a group selected from optionally substituted alkyl, optionally substituted alkenyl, optionally substituted alkinyl, optionally substituted cycloalkyl, optionally substituted aryl, and optionally substituted heterocyclyl.

3. Process according to claim 1 or 2, wherein
X⁻ denotes halide, HSO₄⁻, H₂PO₄⁻ or an anion selected from optionally substituted C₁-C₁₀-alkylsulphonate, optionally substituted C₂-C₁₀-alkenylsulphonate, optionally substituted C₂-C₁₀-alkinylsulphonate, optionally substituted C₃-C₈-cycloalkylsulphonate, optionally substituted C₁-C₁₀-alkylsulphate, optionally substituted C₂-C₁₀-alkenylsulphate, optionally substituted C₂-C₁₀-alkinylsulphate, optionally substituted C₃-C₈-cycloalkylsulphate, optionally substituted C₆-C₁₀-arylsulphonate, optionally substituted C₆-C₁₀-arylsulphate, optionally substituted heterocyclylsulphonate and optionally substituted heterocyclylsulphate;
or
X⁻ denotes R-COO⁻, wherein R is hydrogen, COOH, COO-C₁-C₆-alkyl or a group selected from optionally substituted C₁-C₁₀-alkyl, optionally substituted C₂-C₁₀-alkenyl, optionally substituted C₂-C₁₀-alkinyl, optionally substituted C₃-C₈-cycloalkyl, optionally substituted C₆-C₁₀-aryl, and optionally substituted heterocyclyl.

4. Process according to one of claims 1 to 3, wherein
X⁻ denotes halide, HSO₄⁻, H₂PO₄⁻ or an anion selected from C₁-C₁₀-alkylsulphonate, C₂-C₁₀-alkenylsulphonate, C₂-C₁₀-alkinylsulphonate, C₃-C₈-cycloalkylsulphonate, C₁-C₁₀-alkylsulphate, C₂-C₁₀-alkenylsulphate, C₁-C₁₀-alkinylsulphate, C₃-C₈-cycloalkylsulphate, C₆-C₁₀-arylsulphonate, C₆-C₁₀-arylsulphate, heterocyclylsulphonate and heterocyclylsulphate, which is optionally substituted by one or more non-interfering groups;
or
X⁻ denotes R-COO⁻, wherein R is hydrogen, COOH, COO-C₁-C₆-alkyl or a group selected from C₁-C₁₀-alkyl, C₂-C₁₀-alkenyl, C₂-C₁₀-alkinyl, C₃-C₈-cycloalkyl, C₆-C₁₀-aryl, and heterocyclyl, which is optionally substituted by one or more non-interfering groups.

5. Process according to one of claims 1 to 4, wherein
X⁻ denotes halide, HSO₄⁻, H₂PO₄⁻ or an anion selected from C₁-C₁₀-alkylsulphonate, C₂-C₁₀alkenylsulphonate, C₂-C₁₀-alkinylsulphonate, C₃-C₈-cycloalkylsulphonate, C₁-C₁₀-alkylsulphate, C₂-C₁₀-alkenylsulphate, C₂-C₁₀-alkinylsulphate, C₃-C₈-cycloalkylsulphate, C₆-C₁₀-arylsulphonate, C₆-C₁₀-arylsulphate, heterocyclylsulphonate and beterocyclylsulphate, which is optionally substituted by one or more non-interfering groups which are preferably selected from halogen, OH, =O, CN, NO₂, NH₂, COOH, COO-C₂-C₆-alkyl, C₁-C₆-alkyl, C₁-C₆-haloalkyl, C₁-C₆-alkyloxy, C₃-C₆-cycloalkyl, C₆-C₁₀-aryl;
or
X⁻ denotes R-COO⁻, wherein R is hydrogen, COOH, COO-C₁-C₆-alkyl or a group selected from C₁-C₁₀-alkyl, C₂-C₁₀-alkenyl, C₂-C₁₀-alkinyl, C₃-C₈-cycloalkyl, C₆-C₁₀-aryl, and heterocyclyl, which is optionally substituted by one or more non-interfering groups which are preferably selected from halogen, OH, =O, CN, NO₂, NH₂, COOH, COO-C₁-C₆-alkyl, C₁-C₆-alkyl, C₁-C₆-haloalkyl, C₁-4-alkyloxy, C₃-C₈-cycloalkyl, C₆-C₁₀-aryl.

6. Process according to one of claims 1 to 5, wherein the suitable solvents include water or organic solvents, preferably water or polar organic solvents that are suitable to dissolve tiotropium bicarbonate **2.**

7. Process according to one of claims 1 to 6, wherein the suitable solvents are protic solvents such as alcohols (for example methanol, ethanol, isopropanol) and water, preferably water of pH 2-6 as well as polar organic solvents selected from the group consisting of alcohols such as for example ethyleneglycol and diethyleneglycol, amides such as for example dimethylformamide and N-methyl-pyrrolidinone, ethers such as for example tetrahydrofuran, dioxane, dimethylether and nitriles such as for example acetonitrile.

8. Process according to one of claims 1 to 7, wherein the tiotropium bicarbonate **2** solution is treated with HX at a pH below 5 wherein X may have the meanings specified herein.

9. Process according to one of claims 1 to 8, wherein the process is conducted using a suitable ion exchange resin.

10. Solutions containing a compound of formula **2** 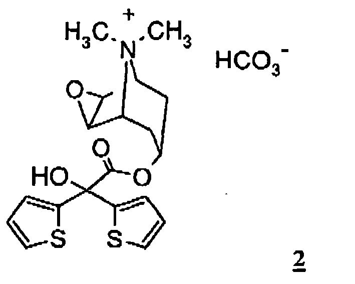 optionally in the form of the solvates or hydrates thereof dissolved or suspended in a suitable solvent, wherein the solvent is selected from among alcohols, water, amides, ethers and nitriles.

11. Solutions according to claim 10 wherein compound **2** is dissolved.

12. Use of bicarbonate **2** 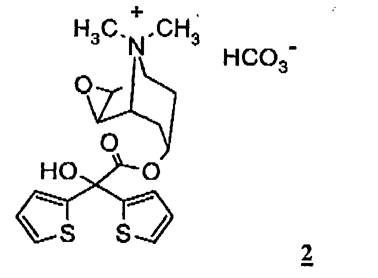 optionally in the form of the solvates or hydrates thereof as a starting material for the preparation of tiotropium salts **1.**

## Patentansprüche

1. Verfahren zur Herstellung neuer Tiotropiumsalze der Formel **1** 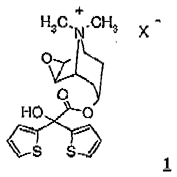 wobei
X⁻ ein Anion bezeichnet, das von HCO₃⁻ (=Bicarbonat) verschieden ist,
**dadurch gekennzeichnet, dass** das Tiotropiumbicarbonat der Formel 2 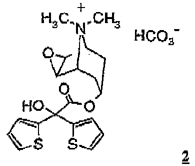 in einem geeigneten Lösungsmittel mit einer Säure HX umgesetzt wird, wobei X die oben angegebenen Bedeutungen aufweist

2. Verfahren nach Anspruch 1, wobei
X⁻ bezeichnet: Halogenid, HSO₄⁻, H₂PO₄⁻ oder ein Anion, ausgewählt aus gegebenenfalls substituiertem Alkylsulfonat, gegebenenfalls substituiertem Alkenylsulfonat, gegebenenfalls substituiertem Alkinylsulfonat, gegebenenfalls substituiertem Cycloalkylsulfonat, gegebenenfalls substituiertem Alkylsulfat, gegebenenfalls substituiertem Alkenylsulfat, gegebenenfalls substituiertem Alkinylsulfat, gegebenenfalls substituiertem Cycloalkylsulfat, gegebenenfalls substituiertem Arylsulfonat, gegebenenfalls substituiertem Arylsulfat, gegebenenfalls substituiertem Heterocyclylsulfonat und gegebenenfalls substituiertem Heterocyclylsulfat;
oder
X⁻ bezeichnet R-COO⁻, wobei R Wasserstoff, COOH, COO-C₁-C₆-Alkyl darstellt oder eine Gruppe, ausgewählt aus gegebenenfalls substituiertem Alkyl, gegebenenfalls substituiertem Alkenyl, gegebenenfalls substituiertem Alkinyl, gegebenenfalls substituiertem Cycloalkyl, gegebenenfalls substituiertem Aryl und gegebenenfalls substituiertem Heterocyclyl.

3. Verfahren nach Anspruch 1 oder 2, wobei
X⁻ bezeichnet: Halogenid, HSO₄⁻, H₂PO₄⁻ oder ein Anion, ausgewählt aus gegebenenfalls substituiertem C₁-C₁₀-Alkylsulfonat, gegebenenfalls substituiertem C₂-C₁₀-Alkenylsulfonat, gegebenenfalls substituiertem C₂-C₁₀-Alkinylsulfonat, gegebenenfalls substituiertem C₃-C₈-Cycloalkylsulfonat, gegebenenfalls substituiertem C₁-C₁₀-Alkylsulfat, gegebenenfalls substituiertem C₂-C₁₀-Alkenylsulfat, gegebenenfalls substituiertem C₂-C₁₀-Alkinylsulfat, gegebenenfalls substituiertem C₃-C₈-Cycloalkylsulfat, gegebenenfalls substituiertem C₆-C₁₀-Arytsulfonat, gegebenenfalls substituiertem C₆-C₁₀-Arylsulfat, gegebenenfalls substituiertem Heterocyclylsulfonat und gegebenenfalls substituiertem Heterocyctylsulfat;
oder
X⁻ bezeichnet R-COO⁻, wobei R Wasserstoff, COOH, COO-C₁-C₆-Alkyl darstellt oder eine Gruppe, ausgewählt aus gegebenenfalls substituiertem C₁-C₁₀-Alkyl, gegebenenfalls substituiertem C₂-C₁₀-Alkenyl, gegebenenfalls substituiertem C₂-C₁₀-Alkinyl, gegebenenfalls substituiertem C₃-C₈-Cycloalkyl, gegebenenfalls substituiertem C₆-C₁₀-Aryl und gegebenenfalls substituiertem Heterocyclyl.

4. Verfahren nach einem der Ansprüche 1 bis 3, wobei
X⁻ bezeichnet: Halogenid, HSO₄⁻, H₂PO₄⁻ oder ein Anion, ausgewählt aus C₁-C₁₀-Alkyl-sulfonat, C₂-C₁₀-Alkenylsulfonat, C₂-C₁₀-Alkinylsulfonat, C₃-C₈-Cycloalkylsulfonat, C₁-C₁₀-Alkylsulfat, C₂-C₁₀-Alkenylsulfat, C₂-C₁₀-Alkinylsulfat, C₃-C₈-Cycloalkylsulfat, C₆-C₁₀-Arylsulfonat, C₆-C₁₀-Arylsulfat, Heterocyclylsulfonat und Heterocyclylsulfat, welches gegebenenfalls substituiert ist mit ein oder mehr nichtstörenden Gruppen;
oder
X⁻ bezeichnet R-COO⁻, wobei R Wasserstoff, COOH, COO-C₁-C₆-Alkyl darstellt oder eine Gruppe, ausgewählt aus C₁-C₁₀-Alkyl, C₂-C₁₀-Alkenyl, C₂-C₁₀-Alkinyl, C₃-C₈-Cycloalkyl, C₆-C₁₀-Aryl und Heterocyclyl, welche gegebenenfalls substituiert ist mit ein oder mehr nicht-störenden Gruppen.

5. Verfahren nach einem der Ansprüche 1 bis 4, wobei
X⁻ bezeichnet: Halogenid, HSO₄⁻, H₂PO₄⁻ oder ein Anion, ausgewählt aus C₁-C₁₀-Alkylsulfonat, C₂-C₁₀-Alkenylsulfonat, C₂-C₁₀-Alkinylsulfonat, C₃-C₈-Cycloalkylsulfonat, C₁-C₁₀-Alkylsulfat, C₂-C₁₀-Alkenylsulfat, C₂-C₁₀-Alkinylsulfat, C₃-C₈-Cycloalkylsulfat, C₆-C₁₀-Arylsufonat, C₆-C₁₀-Arylsulfat, Heterocyclylsulfonat und Heterocyclylsulfat, welches gegebenenfalls substituiert ist mit ein oder mehr nicht-störenden Gruppen, die bevorzugt ausgewählt sind aus Halogen, OH, =O, CN, NO₂, NH₂, COOH, COO-C₁-C₆-Alkyl, C₁-C₆-Alkyl, C₁-C₆-Haloalkyl, C₁-C₆-Alkyloxy, C₃-C₈-Cycloalkyl, C₆-C₁₀-Aryl;
oder
X⁻ bezeichnet R-COO⁻, wobei R Wasserstoff, COOH, COO-C₁-C₆-Alkyl darstellt oder eine Gruppe, ausgewählt aus C₁-C₁₀-Alkyl, C₂-C₁₀-Alkenyl, C₂-C₁₀-Alkinyl, C₃-C₈-Cycloalkyl, C₆-C₁₀-Aryl und Heterocyclyl, welches gegebenenfalls substituiert ist mit ein oder mehr nicht-störenden Gruppen, die bevorzugt ausgewählt sind aus Halogen, OH, =O, CN, NO₂, NH₂, COOH, COO-C₁-C₆-Alkyl, C₁-C₆-Alkyl, C₁-C₆-Haloalkyl, C₁-C₆-Alkyloxy, C₃-C₈-Cycloakyl, C₆-C₁₀-Aryl.

6. Verfahren nach einem der Ansprüche 1 bis 5, wobei die geeigneten Lösungsmittel Wasser oder organische Lösungsmittel umfassen, bevorzugt Wasser oder polare organische Lösungsmittel, die geeignet sind, um Tiotropiumbicarbonat **2** zu lösen.

7. Verfahren nach einem der Ansprüche 1 bis 6, wobei die geeigneten Lösungsmittel protische Lösungsmittel darstellen, wie Alkohole (z.B. Methanol, Ethanol, Isopropanol) und Wasser, bevorzugt Wasser mit einem pH-Wert von 2 bis 6, sowie polare organische Lösungsmittel, ausgewählt aus der Gruppe, bestehend aus Alkoholen, wie beispielsweise Ethylenglykol und Diethylenglykol, Amide, wie beispielsweise Dimethylformamid und N-Methyl-pyrrolidinon, Ether, wie beispielsweise Tetrahydrofuran, Dioxan, Dimethylether, und Nitrile, wie beispielsweise Acetonitril.

8. Verfahren nach einem der Ansprüche 1 bis 7, wobei die Lösung von Tiotropiumbicarbonat **2** bei einem pH-Wert unterhalb 5 mit HX behandelt wird, wobei X die hier angegebenen Bedeutungen aufweist.

9. Verfahren nach einem der Ansprüche 1 bis 8, wobei das Verfahren unter Verwendung eines geeigneten Ionenaustauscherharzes durchgeführt wird.

10. Lösungen, enthaltend eine Verbindung der Formel **2** 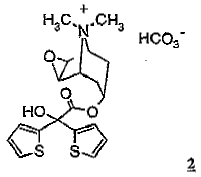 gegebenenfalls in Form der Solvate oder Hydrate hiervon, gelöst oder suspendiert in einem geeigneten Lösungsmittel, wobei das Lösungsmittel ausgewählt ist aus Alkoholen, Wasser, Amiden, Ethern und Nitrilen.

11. Lösungen nach Anspruch 10, worin die Verbindung **2** gelöst ist

12. Verwendung des Bicarbonats **2** 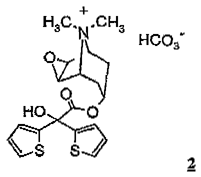 gegebenenfalls in Form der Solvate oder Hydrate hiervon, als Ausgangsmaterial zur Herstellung der Tiotropiumsalze **1**.

## Revendications

1. Procédé de préparation de nouveaux sels de tiotropium de formule 1 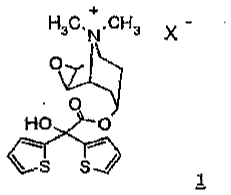 dans laquelle
X⁻ représente un anion qui est différent de HCO₃⁻ (= bicarbonate)
**caractérisé en ce que** le bicarbonate de tiotropium de formule 2 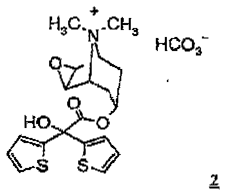 est mis à réagir dans un solvant approprié avec un acide HX où X peut avoir les significations données ci-dessus.

2. Procédé selon la revendication 1, dans lequel
X⁻ représente un halogénure, HSO₄⁻, H₂PO₄⁻ ou un anion choisi parmi un sulfonate d'alkyle éventuellement substitué, un sulfonate d'alcényle éventuellement substitué, un sulfonate d'alcynyle éventuellement substitué, un sulfonate de cycloalkyle éventuellement substitué, un sulfate d'alkyle éventuellement substitué, un sulfate d'alcényle éventuellement substitué, un sulfate d'alcynyle éventuellement substitué, un sulfate de cycloalkyle éventuellement substitué, un sulfonate d'aryle éventuellement substitué, un sulfate d'aryle éventuellement substitué, un sulfonate d'hétérocyclyle éventuellement substitué et un sulfate d'hétérocyclyle éventuellement substitué ;
ou
X⁻ représente R-COO⁻, où R représente un atome d'hydrogène, un groupe COOH, COO-alkyle en C₁ à C₆ ou un groupe choisi parmi les groupes alkyle éventuellement substitué, alcényle éventuellement substitué, alcynyle éventuellement substitué, cycloalkyle éventuellement substitué, aryle éventuellement substitué et hétérocyclyle éventuellement substitué.

3. Procédé selon la revendication 1 ou 2, dans lequel
X⁻ représente un halogénure, HSO₄⁻, H₂PO₄⁻ ou un anion choisi parmi un sulfonate d'alkyle en C₁ à C₁₀ éventuellement substitué, un sulfcnate d'alcényle en C₂ à C₁₀ éventuellement substitué, un sulfonate d'alcynyle en C₂ à C₁₀ éventuellement substitué, un sulfonate de cycloalkyle en C₃ à C₈ éventuellement substitué, un sulfate d'alkyle en C₁ à C₁₀ éventuellement substitué, un sulfate d'alcényle en C₂ à C₁₀ éventuellement substitué, un sulfate d'alcynyle en C₂ à C₁₀ éventuellement substitué, un sulfate de cycloalkyle en C₃ à C₈ éventuellement substitué, un sulfonate d'aryle en C₆ à C₁₀ éventuellement substitué, un sulfate d'aryle en C₆ à C₁₀ éventuellement substitué, un sulfonate d'hétérocyclyle éventuellement substitué et un sulfate d'hétérocyclyle éventuellement substitué ;
ou
X⁻ représente R-COO⁻, où R représente un atome d'hydrogène, un groupe COOH, COO-alkyle en C₁ à C₆ ou un groupe choisi parmi les groupes alkyle en C₁ à C₁₀ éventuellement substitué, alcényle en C₂ à C₁₀ éventuellement substitué, alcynyle en C₂ à C₁₀ éventuellement substitué, cycloalkyle en C₃ à C₈ éventuellement substitué, aryle en C₆ à C₁₀ éventuellement substitué et hétérocyclyle éventuellement substitué.

4. Procédé selon l'une quelconque des revendications 1 à 3, dans lequel
X⁻ représente un halogénure, HSO₄⁻, H₂PO₄⁻ ou un anion choisi parmi un sulfonate d'alkyle en C₁ à C₁₀, un sulfonate d'alcényle en C₂ à C₁₀, un sulfonate d'alcynyle en C₂ à C₁₀, un sulfonate de cycloalkyle en C₃ à C₈, un sulfate d'alkyle en C₁ à C₁₀, un sulfate d'alcényle en C₂ à C₁₀, un sulfate d'alcynyle en C₂ à C₁₀, un sulfate de cycloalkyle en C₃ à C₈, un sulfonate d'aryle en C₆ à C₁₀, un sulfate d'aryle en C₆ à C₁₀, un sulfonate d'hétérocyclyle et un sulfate d'hétérocyclyle, qui est éventuellement substitué par un ou plusieurs groupes non interférants ;
ou
X⁻ représente R-COO⁻, où R représente un atome d'hydrogène, un groupe COOH, COO-alkyle en C₁ à C₆ ou un groupe choisi parmi les groupes alkyle en C₁ à C₁₀, alcényle en C₂ à C₁₀, alcynyle en C₂ à C₁₀, cycloalkyle en C₃ à C₈, aryle en C₆ à C₁₀ et hétérocyclyle, qui est éventuellement substitué par un ou plusieurs groupes non interférants.

5. X⁻ représente un halogénure, HSO₄⁻, H₂PO₄⁻ ou un anion choisi parmi un sulfonate d'alkyle en C₁ à C₁₀, un sulfonate d'alcényle en C₂ à C₁₀, un sulfonate d'alcynyle en C₂ à C₁₀, un sultonate de cycloalkyle en C₃ à C₈, un sulfate d'alkyle en C₁ à C₁₀, un sulfate d'alcényle en C₂ à C₁₀, un sulfate d'alcynyle en C₂ à C₁₀, un sulfate de cycloalkyle en C₃ à C₈, un sulfonate d'aryle en C₆ à C₁₀, un sulfate d'aryle en C₆ à C₁₀, un sulfonate d'hétérocyclyle et un sulfate d'hétérocyclyle, qui est éventuellement substitué par un ou plusieurs groupes non interférants qui sont de préférence choisis parmi un atome d'halogène, les groupes OH, =O, CN, NO₂, NH₂, COOH, COO-alkyle en C₁ à C₆, alkyle en C₁ à C₆, halogénoalkyle en C₁ à C₆, alkyloxy en C₁ à C₆, cycloalkyle en C₃ à C₈, aryle en C₆ à C₁₀ ;
ou
X⁻ représente R-COO⁻, où R représente un atome d'hydrogène, un groupe COOH, COO-alkyle en C₁ à C₆ ou un groupe choisi parmi les groupes alkyle en C₁ à C₁₀, alcényle en C₂ à C₁₀, alcynyle en C₂ à C₁₀, cycloalkyle en C₃ à C₈, aryle en C₆ à C₁₀ et hétérocyclyle, qui est éventuellement substitué par un ou plusieurs groupes non interférants qui sont de préférence choisie parmi un atome d'halogène, les groupes OH, =O, CN, NO₂, NH₂, COOH, COO-alkyle en C₁ à C₆, alkyle en C₁ à C₅, halogénoalkyle en C₁ à C₆, alkyloxy en C₁ à C₆, cycloalkyle en C₃ à C₈, aryle en C₆ à C₁₀.

6. Procédé selon l'une quelconque des revendications 1 à 5, dans lequel les solvants appropriés comprennent l'eau ou des solvants organiques, de préférence l'eau ou des solvants organiques polaires qui sont appropriés pour dissoudre le bicarbonate de tiotropium 2.

7. Procédé selon l'une quelconque des revendications 1 à 6, dans lequel les solvants appropriés sont des solvants protiques tels que des alcools (par exemple, le méthanol, l'éthanol, l'isopropanol) et l'eau, de préférence l'eau de pH 2 à 6 ainsi que des solvants organiques polaires choisis dans le groupe constitué par des alcools tels que, par exemple, l'éthylène glycol et le diéthylène glycol, des amides tels que, par exemple, le diméthylformamide et la N-méthyl-pyrrolidinone, des éthers tels que, par exemple, le tétrahydrofurane, le dioxane, l'éther diméthylique et des nitriles tels que, par exemple, l'acétonitrile.

8. Procédé selon l'une quelconque des revendications 1 à 7, dans lequel la solution de bicarbonate de tiotropium 2 est traitée avec HX à un pH inférieur à 5, où X peut avoir les significations spécifiées dans ce document.

9. Procédé selon l'une quelconque des revendications 1 à 8, dans lequel le procédé est conduit en utilisant une résine échangeuse d'ions appropriée.

10. Solutions contenant un composé de formule 2 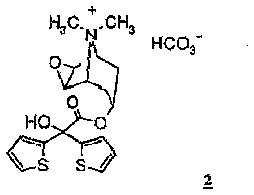 éventuellement sous la forme des solvates ou des hydrates de celui-ci dissous ou en suspension dans un solvant approprié, où le solvant est choisi parmi des alcools, l'eau, des amides, des éthers et des nitriles.

11. Solutions selon la revendication 10, dans lesquelles le composé 2 est dissous.

12. Utilisation du bicarbonate 2
